# EUROPÄISCHE PATENTANMELDUNG

(11) **EP 4 517 102 A1**
(43) Veröffentlichungstag der Anmeldung: **05.03.2025**
(21) Anmeldenummer: 24195149.0
(22) Anmeldetag: 19.08.2024
(51) Int. Cl.: F04D 29/08, F04D 13/06, F04D 29/42, F04D 29/62, A61M 60/232

(54) **PUMPENEINHEIT FÜR EINE ZENTRIFUGALPUMPE SOWIE ZENTRIFUGALPUMPE**

(30) Priorität: 28.08.2023 EP 23193754
(71) Anmelder: Levitronix GmbH, 8048 Zürich (CH)
(72) Erfinder: Hu, Rennan, 8057 Zürich (CH); Willi, Urs, 8590 Romanshorn (CH); Schmid, Alexander, 8915 Hausen am Albis (CH); Barletta, Natale Dr., 8048 Zürich (CH)
(74) Vertreter: IPS Irsch AG

(57) **Zusammenfassung**

Es wird eine Pumpeneinheit für eine Zentrifugalpumpe zum Fördern eines Fluids vorgeschlagen, umfassend ein Pumpengehäuse (2) mit einem Einlass (21) und mit einem Auslass (22) für das Fluid, und einen in dem Pumpengehäuse (2) angeordneten Rotor (10) zum Fördern des Fluids, der um eine axiale Richtung (A) rotierbar ist, wobei die Pumpeneinheit für eine berührungslos magnetische Lagerung des Rotors (10) und für einen berührungslos magnetischen Antrieb des Rotors (10) ausgestaltet ist, wobei das Pumpengehäuse (2) ein Bodenteil (3) und einen Deckel (4) zum Verschliessen des Bodenteils (3) aufweist, wobei das Bodenteil (3) einen zylindrischen Becher (31) zur Aufnahme des Rotors (10) und eine im Wesentlichen ringförmige erste Dichtfläche (5) zum dichtenden Zusammenwirken mit dem Deckel (4) aufweist, und wobei der Deckel (4) eine im Wesentlichen ringförmige zweite Dichtfläche (6) aufweist, die zum dichtenden Zusammenwirken mit der ersten Dichtfläche (5) ausgestaltet ist. Eine der beiden Dichtflächen (5, 6) ist als Rippenfläche mit mindestens einer radialen Dichtungsrippe (7) ausgestaltet, die sich in Umfangsrichtung entlang der gesamten Dichtfläche (5, 6) erstreckt, während die andere der beiden Dichtflächen (6, 5) als glatte Fläche ausgestaltet ist. Ferner wird eine Zentrifugalpumpe zum Fördern eines Fluids vorgeschlagen, die eine solche Pumpeneinheit umfasst.

## Beschreibung

Die Erfindung betrifft eine Pumpeneinheit für eine Zentrifugalpumpe zum Fördern eines Fluids gemäss dem Oberbegriff des unabhängigen Patentanspruchs. Die Erfindung betrifft ferner eine Zentrifugalpumpe mit einer solchen Pumpeneinheit.

Es sind Zentrifugalpumpen bekannt, welche eine Pumpeneinheit und eine Antriebseinheit umfassen, wobei in der Pumpeneinheit ein Rotor vorgesehen ist, welcher das Flügelrad der Zentrifugalpumpe bildet. In der Antriebseinheit ist ein Stator vorgesehen. Dabei ist der Rotor in der Pumpeneinheit mittels der Antriebseinheit berührungslos magnetisch lagerbar und berührungslos zur Rotation um eine axiale Richtung antreibbar. Solche Zentrifugalpumpen werden beispielsweise von der Anmelderin unter der Produktbezeichnung Levitronix^{®} BPS Pumpen vertrieben.

Der Stator und der Rotor bilden einen elektromagnetischen Drehantrieb. Bei den Levitronix^{®} BPS Pumpen, beispielsweise, ist der elektromagnetische Drehantrieb nach dem Prinzip des lagerlosen Motors ausgestaltet. Mit dem Begriff lagerloser Motor ist dabei ein elektromagnetischer Drehantrieb gemeint, bei welchem der Rotor vollkommen magnetisch bezüglich des Stators lagerbar ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine durch die axiale Richtung definierte Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Es sind natürlich auch andere Ausgestaltungen von Zentrifugalpumpen bekannt, bei welchen der Rotor berührungslos magnetisch gelagert ist, beispielsweise solche bei denen separate Magnetlager für den Rotor vorgesehen sind, sodass die magnetische Lagerfunktion von der Antriebsfunktion getrennt ist. Beispielsweise sind dazu separate Spulen vorgesehen, mit denen nur die Lagerkräfte für den Rotor realisiert werden, die aber keinen Beitrag zum Antrieb des Rotors leisten.

Zentrifugalpumpen mit berührungslos magnetisch gelagerten und angetriebenen Rotoren, beispielsweise solche, die nach dem Prinzip des lagerlosen Motors ausgestaltet und betrieben werden, haben sich in einer Vielzahl von Anwendungen bewährt. Aufgrund der Abwesenheit von mechanischen Lagern eignen sich solche Zentrifugalpumpen für Anwendungen, bei denen sehr empfindliche Substanzen gefördert werden, beispielsweise Blutpumpen, oder bei denen sehr hohe Anforderungen an die Reinheit gestellt werden, beispielsweise in der Halbleiterindustrie, der pharmazeutischen Industrie, der biotechnologischen Industrie, oder bei denen abrasive oder aggressive Substanzen gefördert werden, welche mechanische Lager sehr schnell zerstören würden, beispielsweise Pumpen für Slurry, Schwefel-, Phosphorsäure oder andere Chemikalien in der Halbleiterindustrie.

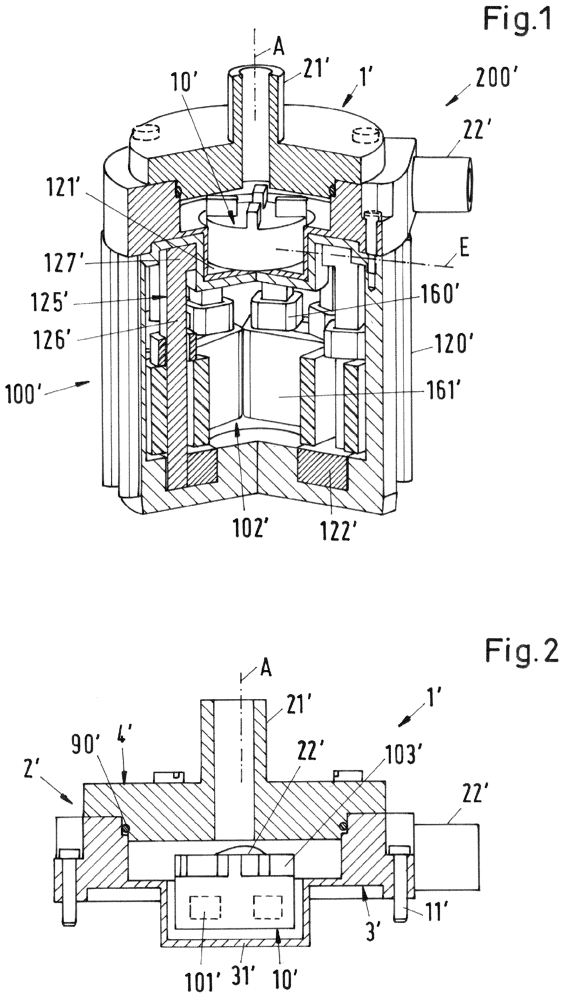

Fig. 1 zeigt eine Darstellung einer aus dem Stand der Technik bekannten Zentrifugalpumpe, die nach dem Prinzip des lagerlosen Motors ausgestaltet ist. Es handelt sich beispielsweise um eine Levitronix^{®} BPS Pumpe. Zum besseren Verständnis ist in Fig. 1 ein Segment herausgeschnitten, damit das Innere der Zentrifugalpumpe sichtbar ist.

Um anzuzeigen, dass es sich bei der Darstellung in Fig. 1 und in Fig. 2 um eine Vorrichtung aus dem Stand der Technik handelt, sind hier die Bezugszeichen jeweils mit einem Hochkomma bzw. mit einem Strich versehen. Die Zentrifugalpumpe ist gesamthaft mit dem Bezugszeichen 200' bezeichnet.

Die Zentrifugalpumpe 200' umfasst eine Antriebseinheit 100` und eine Pumpeneinheit 1'. Zum besseren Verständnis ist in Fig. 2 die Pumpeneinheit 1' in einer Schnittdarstellung gezeigt, wobei der Schnitt in axialer Richtung A erfolgt.

In der Pumpeneinheit 1' ist ein Rotor 10' angeordnet, welcher das Flügelrad bzw. das Laufrad bildet, mit dem das Fluid gefördert wird. Die Antriebseinheit 100' umfasst ein Motorgehäuse 120`, in welchem ein Stator 102` angeordnet ist, der mit dem Rotor 10` einen elektromagnetischen Drehantrieb zum Rotieren des Rotors um eine axiale Richtung A bildet. Die Antriebseinheit 100' ist zur berührungslosen magnetischen Lagerung des Rotors 10` nach dem Prinzip des lagerlosen Motors ausgestaltet. Hierzu ist der Stator 102` als Lager- und Antriebsstator ausgestaltet, mit welchem der Rotor 10' berührungslos magnetisch zur Rotation um die axiale Richtung A antreibbar und berührungslos magnetisch bezüglich des Stators 102` lagerbar ist, wobei der Rotor 10` bezüglich der axialen Richtung A passiv magnetisch stabilisiert ist, und in einer zur axialen Richtung A senkrechten radialen Ebene, die in Fig. 1 durch die Linie E angedeutet ist, aktiv magnetisch gelagert ist. Das Motorgehäuse 120' weist in einer seiner axialen Stirnflächen, nämlich in der darstellungsgemäss oberen, eine Ausnehmung 121' auf, in welche die Pumpeneinheit 1' einsetzbar ist.

Der elektromagnetische Drehantrieb mit dem Stator 102' und dem Rotor 10` ist als sogenannter Tempelmotor ausgestaltet. Der Stator 102` umfasst eine Mehrzahl von Spulenkernen 125`, hier acht Spulenkerne 125`, von denen jeder einen Längsschenkel 126` umfasst, welcher sich von einem ersten Ende, in Fig. 1 das darstellungsgemäss untere Ende, in axialer Richtung A bis zu einem zweiten Ende erstreckt, sowie einen Querschenkel 127', welcher an dem zweiten Ende des Längsschenkels 126` und in der radialen Ebene E angeordnet ist. Jeder Querschenkel 127` erstreckt sich von dem zugehörigen Längsschenkel 126` in radialer Richtung auf den Rotor 10' zu und wird durch eine radial innenliegende Stirnfläche begrenzt. Die Spulenkerne 126` sind bezüglich der Umfangsrichtung um den Rotor 10` herum angeordnet, sodass der Rotor 10` zwischen den radial innenliegenden Stirnflächen der Querschenkel 127` der Spulenkerne 126` angeordnet ist.

Alle ersten Enden der Längsschenkel 126` sind durch einen Rückschluss 122' zur Führung des magnetischen Flusses miteinander verbunden. An jedem Längsschenkel 126` ist mindestens eine konzentrierte Wicklung 160', 161' vorgesehen, welche den jeweiligen Längsschenkel 126` umgibt. Bezüglich Anzahl und Anordnung der konzentrierten Wicklungen 160', 161' sind zahlreiche Varianten bekannt, die hier nicht näher erläutert werden. Es gibt beispielsweise solche Wicklungen 160`, die um genau einen Längsschenkel 126` herum gewickelt sind, und solche Wicklungen 161', die um genau zwei Längsschenkel 126` herum angeordnet sind.

Die Mehrzahl der Längsschenkel 126', die sich in axialer Richtung A erstrecken und an die Säulen eines Tempels erinnern, hat dem Tempelmotor seinen Namen gegeben.

Die Pumpeneinheit 1' umfasst ein Pumpengehäuse 2' mit einem Einlass 21' und mit einem Auslass 22' für das Fluid, sowie den in dem Pumpengehäuse 2' angeordneten Rotor 10' zum Fördern des Fluids, der um die axiale Richtung A rotierbar ist. Der Rotor 10` umfasst einen magnetisch wirksamen Kern 101', welcher für die Drehmomentbildung sowie für die Erzeugung der magnetischen Lagerkräfte magnetisch mit dem Stator 102' zusammenwirkt.

Der magnetisch wirksame Kern 101' ist beispielsweise ein permanentmagnetischer Ring oder eine permanentmagnetische Scheibe.

Es sind auch solche Ausgestaltungen möglich, bei denen der magnetisch wirksame Kern 101' permanentmagnetfrei, also ohne Permanentmagnete ausgestaltet ist. Der Rotor 10` ist dann z.B. als Reluktanzläufer ausgestaltet. Der magnetisch wirksame Kern 101' des Rotors 10` besteht dann beispielsweise aus einem weichmagnetischen Material. Geeignete weichmagnetische Materialien für den magnetisch wirksamen Kern 101 ` sind beispielsweise ferromagnetische oder ferrimagnetische Materialien, also insbesondere Eisen, Nickel-Eisen, Kobalt-Eisen, Silizium-Eisen, Mu-Metall.

Ferner sind Ausgestaltungen möglich, bei denen der magnetisch wirksame Kern 101' des Rotors 10' sowohl ferromagnetische Materialien als auch permanentmagnetische Materialien umfasst. Beispielsweise können Permanentmagnete in einen ferromagnetischen Grundkörper eingelegt bzw. eingesetzt werden. Solche Ausgestaltungen sind z.B. vorteilhaft, wenn man bei grossen Rotoren die Kosten durch Einsparen von permanentmagnetischem Material reduzieren will.

Typischerweise ist der magnetisch wirksame Kern 101' mit einem Kunststoff vollständig ummantelt. In anderen Ausführungsformen ist der magnetisch wirksame Kern 101 ` vollständig in einer Ummantelung eingeschlossen, die aus einem keramischen Material oder aus einem metallischen Material, beispielsweise rostfreier Stahl oder Titan oder Tantal, besteht.

Der Rotor 10' umfasst ferner eine Mehrzahl von Flügeln 103' zum Fördern des Fluids vom Einlass 21' zum Auslass 22'.

Das Pumpengehäuse 2' umfasst ein Bodenteil 3' und einen Deckel 4' zum Verschliessen des Bodenteils 3`, wobei zwischen dem Bodenteil 3' und dem Deckel 4' ein Dichtungselement 90' vorgesehen ist, beispielsweise ein O-Ring oder eine Flachdichtung, um eine Leckage des Fluids in die Umgebung zu vermeiden.

Das Bodenteil 3' des Pumpengehäuses 2' weist einen zylindrischen Becher 31' zur Aufnahme des Rotors 10` auf. Der Becher 31' wird in die Ausnehmung 121' im Motorgehäuse 120' eingesetzt, sodass der Rotor 10', genauer gesagt der magnetisch wirksame Kern 101' des Rotors 10` zwischen den Querschenkeln 127` der Spulenkerne 126` angeordnet ist.

Die Pumpeneinheit 1' ist beispielsweise mittels einer Mehrzahl von Schrauben 11' am Motorgehäuse 120' befestigt.

Für viele Anwendungen, beispielsweise für Anwendungen in der Halbleiterindustrie, wird die Pumpeneinheit 1' - mit Ausnahme des magnetisch wirksamen Kerns 101' - aus einem Kunststoff gefertigt, beispielsweise aus einem Perfluoralkoxy-Polymer (PFA) oder aus Polytetrafluorethylen (PTFE), weil dies Kunststoffe mit einer besonders hohen chemischen Resistenz sind. Diese Kunststoffe sind praktisch inerte Stoffe, die auch von chemisch sehr aggressiven Substanzen, wie sie häufig in der Halbleiterindustrie zum Einsatz kommen, nicht angegriffen werden können. Zudem sind PFA und PTFE sehr reine Kunststoffe, weil sie üblicherweise keine Zusatzstoffe aufweisen und ihre Molekülkomplexe zumindest näherungsweise inert sind. PFA ist häufig bevorzugt, weil es in Spritzgiessverfahren verarbeitet werden kann.

Das Dichtungselement 90' zur Abdichtung zwischen dem Bodenteil 3' und dem Deckel 4' ist beispielsweise als O-Ring oder als ringförmige Flachdichtung ausgestaltet. Für das Dichtungselement 90` werden Elastomere bevorzugt, insbesondere auch deshalb, weil Elastomere sehr gute Rückstellkräfte aufweisen. In der Halbleiterindustrie, wo extrem hohe Anforderungen an die Reinheit gestellt werden, ist es auch üblich, Perfluorelastomere (Perfluorkautschuk, FFPM) für das Dichtungselement 90' zu verwenden. FFPM wird insbesondere dort eingesetzt, wo eine sehr gute thermische und/oder chemische Beständigkeit notwendig ist.

Trotz dieser sehr modernen und leistungsfähigen Materialien können Probleme mit Leckagen auftreten, beispielsweise in der Halbleiterindustrie. Insbesondere bei hohen Temperaturen kann die chemische Resistenz der Elastomere unzureichend sein, um einen dauerhaften, sicheren Betrieb der Zentrifugalpumpe zu gewährleisten. In der Halbleiterindustrie gibt es beispielsweise Prozesse, bei denen Schwefelsäure mit einer Temperatur von bis zu 220°C gefördert werden muss.

Ein weiters Problem ist das Ausgasen, beispielsweise von Zusätzen in den Dichtungselementen. Beispielsweise in der Halbleiterindustrie können allerkleinste Verunreinigungen verheerende Folgen haben, weil die hergestellten Produkte durch minimale Verunreinigungen unbrauchbar gemacht werden können. Hierbei ist zu bemerken, dass heutzutage in der Halbleiterindustrie Strukturen generiert werden, die im Bereich von fünf Nanometer oder sogar noch kleiner liegen. Daher ist die Reinheit in der Halbleiterindustrie von herausragender Bedeutung.

Ferner kann das Kriechen des Dichtungselements bzw. von Teilen des Dichtungselements zu Problemen führen, weil beispielsweise durch Kriechprozesse bedingt Risse oder andere Beschädigungen in dem Dichtungselement auftreten können.

Speziell bei höheren Temperaturen und/oder bei zyklischen Prozessen mit häufigen Druck-oder Temperaturänderungen besteht die Gefahr von Leckagen, bei denen sehr gefährliche, aggressive oder schädliche Fluide in die Umgebung der Zentrifugalpumpe austreten können.

Ausgehend von diesem Stand der Technik ist es daher eine Aufgabe der Erfindung, eine Pumpeneinheit mit einem berührungslos magnetisch lagerbaren Rotor für eine Zentrifugalpumpe vorzuschlagen, die eine erhöhte Betriebssicherheit, insbesondere gegenüber Leckagen, und im Hinblick auf Reinheit aufweist. Zudem ist es eine Aufgabe der Erfindung, eine Zentrifugalpumpe mit einer solchen Pumpeneinheit vorzuschlagen.

Der diese Aufgabe lösende Gegenstand der Erfindung ist durch die Merkmale des unabhängigen Patentanspruchs gekennzeichnet.

Erfindungsgemäss wird also eine Pumpeneinheit für eine Zentrifugalpumpe zum Fördern eines Fluids vorgeschlagen, umfassend ein Pumpengehäuse mit einem Einlass und mit einem Auslass für das Fluid, und einen in dem Pumpengehäuse angeordneten Rotor zum Fördern des Fluids, der um eine axiale Richtung rotierbar ist, wobei die Pumpeneinheit für eine berührungslos magnetische Lagerung des Rotors und für einen berührungslos magnetischen Antrieb des Rotors ausgestaltet ist, wobei das Pumpengehäuse ein Bodenteil und einen Deckel zum Verschliessen des Bodenteils aufweist, wobei das Bodenteil einen zylindrischen Becher zur Aufnahme des Rotors und eine im Wesentlichen ringförmige erste Dichtfläche zum dichtenden Zusammenwirken mit dem Deckel aufweist, und wobei der Deckel eine im Wesentlichen ringförmige zweite Dichtfläche aufweist, die zum dichtenden Zusammenwirken mit der ersten Dichtfläche ausgestaltet ist. Eine der beiden Dichtflächen ist als Rippenfläche mit mindestens einer radialen Dichtungsrippe ausgestaltet, die sich in Umfangsrichtung entlang der gesamten Dichtfläche erstreckt, während die andere der beiden Dichtflächen als glatte Fläche ausgestaltet ist.

Durch diese Ausgestaltung ist es möglich, auf ein separates Dichtungselement zwischen dem Bodenteil und dem Deckel zu verzichten, welches während des normalen, also störungsfreien Betriebs mit dem Fluid in Kontakt käme. Im normalen, das heisst störungsfreien Betrieb kommt also das zu fördernde Fluid mit keinem separaten Dichtungselement in Berührung, sodass auch keine Gefahr der Verunreinigung des Fluids durch ein solches separates Dichtungselement besteht. Durch den Verzicht auf ein solches separates Dichtungselement 90` (Fig. 2) werden alle diejenigen Probleme gelöst, die in den bekannten Pumpeneinheiten 1' durch dieses separate Dichtungselement 90' verursacht werden.

Der Verzicht auf ein solches separates Dichtungselement stellt eine deutliche Verbesserung im Hinblick auf die Reinheit des zu fördernden Fluids dar. Da das Fluid im normalen Betriebszustand nicht mit einem solchen separaten Dichtungselement in Kontakt kommen kann, besteht auch keine Gefahr, dass das Fluid durch ein solches separates Dichtungselement verunreinigt wird, beispielsweise durch das Austreten von Zusatzstoffen aus dem Dichtungselement, wie es beispielsweise bei Elastomer Dichtungen vorkommen kann.

Vorzugsweise ist ein radiales Verstärkungselement vorgesehen, welches ringförmig ausgestaltet ist und sich radial aussenliegend um die beiden Dichtflächen herum erstreckt. Das radiale Verstärkungselement, welches die beiden Dichtflächen radial aussenliegend umgibt, stabilisiert die erste und die zweite Dichtfläche und ist daher vorteilhaft im Hinblick auf die Verhinderung von Deformationen der Dichtflächen oder Relativbewegungen der beiden Dichtflächen zueinander. Hierdurch ist es in noch höherem Masse gewährleistet, dass sich zwischen den beiden Dichtflächen auch bei höherem Druck im Pumpengehäuse keine Spalte oder sonstige Leckagepfade öffnen. Zudem ist das radiale Verstärkungselement vorteilhaft, um insbesondere auch ein Kriechen des Bodenteils oder des Deckels weiter zu reduzieren oder sogar ganz zu verhindern, insbesondere dann, wenn das Pumpengehäuse aus einem zum Kriechen neigenden Kunststoff besteht, beispielsweise aus PFA oder PTFE.

Vorzugsweise sind in der als Rippenfläche ausgestalteten ersten oder zweiten Dichtfläche mehrere Dichtungsrippen vorgesehen, von denen sich jede vollständig entlang des gesamten Umfangs der Rippenfläche erstreckt. Jede dieser Dichtungsrippen liegt an der als glatte Fläche ausgestalteten zweiten oder ersten Dichtfläche an. Das heisst, jede Dichtungsrippe ist in unmittelbarem körperlichem Kontakt mit der als glatte Fläche ausgestalteten Dichtfläche. Mit dem Begriff "glatte Fläche" ist insbesondere gemeint, dass diese Dichtfläche keine Nuten oder sonstige Ausnehmungen aufweist, in welche die Dichtungsrippen eingreifen können. Die Dichtungsrippen liegen also auf dieser unstrukturierten glatten Fläche auf.

Es sind Ausgestaltungen möglich, bei denen die erste Dichtfläche als die Rippenfläche ausgestaltet ist und die zweite Dichtfläche als die glatte Fläche, das heisst die Dichtungsrippen sind dann an dem Bodenteil vorgesehen, und die zweite Dichtfläche, also die des Deckels, ist unstrukturiert als glatte Dichtfläche ausgestaltet.

Ferner sind Ausgestaltungen möglich, bei denen die zweite Dichtfläche als die Rippenfläche ausgestaltet ist und die erste Dichtfläche als die glatte Fläche, das heisst die Dichtungsrippen sind dann an dem Deckel vorgesehen, und die erste Dichtfläche, also die des Bodenteils, ist unstrukturiert als glatte Dichtfläche ausgestaltet.

Mit dem Begriff "radiale Dichtungsrippe" ist gemeint, dass die Dichtungsrippe eine Erstreckung in der zur axialen Richtung senkrechten radialen Richtung hat. Dadurch können die Dichtungsrippen radiale Kräfte aufnehmen. Dabei ist es nicht notwendig, aber möglich, dass sich die Dichtungsrippen senkrecht zur axialen Richtung erstrecken. Sie können sich auch schräg zur axialen Richtung erstrecken.

In einer bevorzugten Ausgestaltung ist jede Dichtungsrippe jeweils kreisförmig ausgestaltet, das heisst als ein Kreisring, der sich in Umfangsrichtung entlang der gesamten Dichtfläche erstreckt. Diese Ausgestaltung ist zwar bevorzugt, aber nicht notwendig. Es sind auch solche Ausgestaltungen möglich, bei welchen jede Dichtungsrippe beispielsweise oval oder spiralförmig ausgestaltet ist. Die spiralförmige Ausgestaltung ist insbesondere für solche Pumpeneinheiten vorteilhaft, bei welchen das Pumpengehäuse als Spiralgehäuse (Volute) ausgestaltet ist.

Ganz besonders bevorzugt ist jede Dichtungsrippe jeweils als eine geschlossenen Dichtungsrippe ausgestaltet, womit gemeint ist, dass die Dichtungsrippe keinen Anfang und kein Ende aufweist. Beispielsweise ist jede Dichtungsrippe als ein geschlossener Ring ausgestaltet, der bezüglich der Umfangsrichtung kein Anfang und kein Ende hat.

Das Bodenteil und der Deckel sind über einen Presssitz miteinander verbunden, der dadurch realisiert wird, dass jede Dichtungsrippe der als Rippenfläche ausgestalteten ersten oder zweiten Dichtfläche gegen die als glatte Fläche ausgestaltete zweite oder erste Dichtfläche gepresst wird, wodurch die dichtende Verbindung zwischen dem Bodenteil und dem Deckel entsteht. Das Zusammensetzen des Deckels und des Bodenteils erfolgt dadurch, dass der Deckel und das Bodenteil in axialer Richtung relativ zueinander bewegt werden, sodass die erste Dichtfläche und die zweite Dichtfläche gegeneinander gepresst werden, sodass sie dichtend zusammenwirken. Ein Vorteil ist hierbei, dass die Einbaurichtung, in welcher der Deckel und das Bodenteil zusammengesetzt werden, die axiale Richtung ist und die Dichtungsrippen sich in der radialen Richtung, also senkrecht zur Einbaurichtung erstrecken, und nicht in der Einbaurichtung.

Vorzugsweise umfasst die Rippenfläche mehrere radiale Dichtungsrippen, beispielsweise drei Dichtungsrippen, von denen sich jede in Umfangsrichtung entlang der gesamten Rippenfläche erstreckt, wobei die Dichtungsrippen bezüglich der axialen Richtung benachbart zueinander angeordnet sind, und zwischen zwei benachbarten Dichtungsrippen jeweils ein Tal vorgesehen ist, wobei jedes Tal einen von Null verschiedenen radialen Abstand von der glatten Fläche hat, und wobei jede Dichtungsrippe eine in radialer Richtung gemessene Höhe aufweist, die grösser ist als der radiale Abstand des Tals von der glatten Fläche. Durch diese Ausgestaltung lässt sich eine besonders gute Dichtwirkung zwischen dem Bodenteil und dem Deckel realisieren.

Eine bevorzugte Massnahme besteht darin, dass das radiale Verstärkungselement sowohl in den Deckel als auch in das Bodenteil eingreift. Hierdurch kann das radiale Verstärkungselement sowohl das Bodenteil als auch den Deckel verstärken, wodurch unerwünschte Relativbewegungen zwischen dem Bodenteil und dem Deckel, beispielsweise durch Deformationen, oder auch Kriecheffekte weiter reduziert werden können.

Eine weitere bevorzugte Ausgestaltung besteht darin, dass die glatte Fläche kegelstumpfförmig ausgestaltet ist. Wenn beispielsweise die erste Dichtfläche als die glatte Fläche ausgestaltet ist, so kann diese derart kegelstumpfförmig ausgestaltet sein, dass sie sich in Einbaurichtung gesehen verjüngt. Bei einer entsprechenden Ausgestaltung der zweiten Dichtfläche kann dann die Stärke des Presssitzes zwischen dem Bodenteil und dem Deckel dadurch eingestellt werden, wie tief der Deckel bezüglich der axialen Richtung in die erste Dichtfläche hineingeschoben wird. Auch ist ein Nachstellen des Presssitzes nach einer längeren Betriebsdauer möglich. Hierdurch können beispielsweise Langzeit-Kriecheffekte ausgeglichen werden. Ferner ist es einfacher und materialschonender möglich, den Deckel wieder von dem Bodenteil zu trennen, beispielsweise für Wartungsarbeiten.

Ferner ist es bevorzugt, dass bezüglich der radialen Richtung zwischen den beiden Dichtflächen einerseits und dem radialen Verstärkungselement andererseits eine ringförmige Sicherheitsdichtung angeordnet ist, welche im Fehlerfall ein Austreten des Fluids zwischen dem Bodenteil und dem Deckel verhindert. Während des normalen, also störungsfreien Betriebs der Pumpeneinheit kommt diese Sicherheitsdichtung nicht in Kontakt mit dem Fluid und hat auch keine Dichtfunktion. Sollte es aber zu einem Fehlerfall oder einer erheblichen Störung kommen, sodass die Dichtwirkung zwischen der ersten und der zweiten Dichtfläche nicht mehr in ausreichendem Masse gewährleistet ist, so kann diese Sicherheitsdichtung eine Kontamination des Fluids sowie ein Austreten des Fluids wirkungsvoll verhindern. Beispielsweise in der Halbleiterindustrie könnten solche Fehlerfälle katastrophale Folgen haben, weil sie zu Ausschuss von Material, z.B. Wafern, führen können, was sehr hohe Kosten verursachen kann. Ferner kann das Austreten des Fluids, beispielsweise im Falle aggressiver Chemikalien zu einer erheblichen Gefahr für die Umwelt führen.

Die Sicherheitsdichtung kann jedoch auch an anderen Stellen platziert werden, beispielsweise radial aussenliegend bezüglich des radialen Verstärkungselements. Wesentlich ist bei Ausgestaltungen mit der Sicherheitsdichtung lediglich, dass die Sicherheitsdichtung im normalen, also störungsfreien Betrieb nicht in Kontakt mit dem zu fördernden Fluid kommen kann.

Für einige Anwendungen ist es vorteilhaft, dass auf dem Deckel ein axiales Verstärkungselement zum Aufnehmen von in axialer Richtung wirkenden Kräften vorgesehen ist, wobei das axiale Verstärkungselement so angeordnet ist, dass sich der Deckel bezüglich der axialen Richtung zwischen dem axialen Verstärkungselement und dem Bodenteil befindet.

Vorzugsweise ist dann eine Stützstruktur vorgesehen, welche mit dem axialen Verstärkungselement verbindbar ist, und welche so angeordnet ist, dass das Pumpengehäuse bezüglich der axialen Richtung zwischen dem axialen Verstärkungselement und der Stützstruktur eingespannt werden kann. Vorzugsweise ist die Stützstruktur aus einem metallischen Material gefertigt, damit sie eine hohe Stabilität hat. Die Stützstruktur kann beispielsweise als metallischer Montagering ausgestaltet sein, welcher um den zylindrischen Becher des Bodenteils herum angeordnet ist, und welcher fest mit dem axialen Verstärkungselement verbindbar ist, beispielsweise mittels Schrauben.

In einer bevorzugten Ausgestaltung bildet ein Motorgehäuse der Zentrifugalpumpe die Stützstruktur und das axiale Verstärkungselement ist zum Befestigen an dem Motorgehäuse der Zentrifugalpumpe ausgestaltet. Beispielsweise ist das axiale Verstärkungselement mittels Schrauben an dem Motorgehäuse befestigt, sodass das Pumpengehäuse zwischen dem axialen Verstärkungselement und dem Motorgehäuse eingespannt ist.

Das axialen Verstärkungselement ist beispielsweise vorteilhaft, wenn mit der Pumpeneinheit ein hoher Druck generiert werden soll, oder wenn ein Fluid bei hoher Temperatur gefördert werden soll, oder bei grösseren Pumpeneinheiten zum Generieren grosser Durchflüsse und/oder grosser Förderhöhen. Das axiale Verstärkungselement verleiht der Pumpeneinheit eine höhere Stabilität, wodurch insbesondere Kriecheffekten entgegengewirkt werden kann, welche durch axiale Kräfte verursacht werden können.

Das axiale Verstärkungselement umfasst vorzugsweise einen metallischen Grundkörper, der beispielsweise aus einem Edelstahl gefertigt ist, und eine Ummantelung aus einem Kunststoff, mit welcher der Grundkörper gekapselt ist.

Das axiale Verstärkungselement kann auch mit einer Federwirkung ausgestaltet sein, beispielsweise mittels Federscheiben. Hierdurch ist ein Nachstellen der Dichtkräfte zwischen dem ersten und der zweiten Dichtfläche möglich.

Falls die Pumpeneinheit mit dem axialen Verstärkungselement ausgestaltet ist, ist es unter konstruktiven Aspekten eine vorteilhafte Massnahme, dass das axiale Verstärkungselement einstückig mit dem radialen Verstärkungselement ausgestaltet ist.

Gemäss einer weiteren bevorzugten Ausführungsform umfasst das Bodenteil eine ringförmige Nut, die von einer radialen Innenwand und von einer radialen Aussenwand begrenzt wird, wobei der Deckel zum dichtenden Eingreifen in die Nut ausgestaltet ist, und wobei die radiale Innenwand oder die radiale Aussenwand die erste Dichtfläche bildet.

Eine weitere bevorzugte Ausführungsform besteht darin, dass im Deckel eine ringförmige Ausnehmung vorgesehen ist, die sich radial innenliegend und benachbart zu der zweiten Dichtfläche erstreckt, wobei die ringförmige Nut durch einen ringförmigen Steg von der zweiten Dichtfläche getrennt ist, sodass im Betriebszustand der Druck des Fluids auf den Steg das dichtende Zusammenwirken der ersten Dichtfläche und der zweiten Dichtfläche verstärkt. Durch diese Ausgestaltung wird im Betriebszustand der Druck des Fluids im Pumpengehäuse dazu genutzt, eine zusätzliche, radial nach aussen gerichtete Kraft zu generieren, welche die Dichtwirkung zwischen den beiden Dichtflächen vergrössern kann.

Wie bereits gesagt sind solche Ausgestaltungen möglich, bei welchen die erste Dichtfläche als die glatte Dichtfläche ausgestaltet ist, und die zweite Dichtfläche als die Rippenfläche ausgestaltet ist.

Natürlich sind auch Ausgestaltungen möglich, bei welchen die erste Dichtfläche als die Rippenfläche ausgestaltet ist.

Vorzugsweise ist das radiale Verstärkungselement als metallischer Ring ausgestaltet, der mit einer Beschichtung aus einem Kunststoff gekapselt ist. Besonders bevorzugt besteht der metallische Ring aus einem rostfreien Stahl oder aus einem Edelstahl.

Eine besonders bevorzugte Ausgestaltung ist es, dass der Auslass für das Fluid im Bodenteil des Pumpengehäuses angeordnet ist. Das heisst, das Fluid wird vorzugsweise aus dem Bodenteil aus dem Pumpengehäuse abgeführt.

Durch die Erfindung wird ferner eine Zentrifugalpumpe zum Fördern eines Fluids vorgeschlagen mit einer erfindungsgemäss ausgestalteten Pumpeneinheit, und mit einer Antriebseinheit, die ein Motorgehäuse umfasst, in welchem ein Stator angeordnet ist, der mit dem Rotor einen elektromagnetischen Drehantrieb zum Rotieren des Rotors um die axiale Richtung bildet, wobei die Antriebseinheit zur berührungslosen magnetischen Lagerung des Rotors ausgestaltet ist.

Vorzugsweise ist der Stator als Lager- und Antriebsstator ausgestaltet, mit welchem der Rotor berührungslos magnetisch antreibbar und berührungslos magnetisch bezüglich des Stators lagerbar ist, wobei der Rotor bezüglich der axialen Richtung passiv magnetisch stabilisiert ist, und in einer zur axialen Richtung senkrechten radialen Ebene aktiv magnetisch gelagert ist, wobei das Motorgehäuse in einer axialen Stirnfläche eine Ausnehmung aufweist, in welche der zylindrische Becher des Pumpengehäuses einsetzbar ist.

Besonders bevorzugt ist der elektromagnetische Drehantrieb als Tempelmotor ausgestaltet, wobei der Stator eine Mehrzahl von Spulenkernen aufweist, von denen jeder einen Längsschenkel umfasst, welcher sich von einem ersten Ende in axialer Richtung bis zu einem zweiten Ende erstreckt, sowie einen Querschenkel, welcher an dem zweiten Ende des Längsschenkels und in der radialen Ebene angeordnet ist, und welcher sich von dem Längsschenkel in radialer Richtung erstreckt, wobei die Spulenkerne bezüglich der Umfangsrichtung um den Rotor herum angeordnet sind, sodass der Rotor zwischen den Querschenkeln der Spulenkerne angeordnet ist, und wobei an jedem Längsschenkel mindestens eine konzentrierte Wicklung vorgesehen ist, welche den jeweiligen Längsschenkel umgibt.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der schematischen Zeichnung zeigen (teilweise im Schnitt):
- Fig. 1:: eine perspektivische Darstellung einer Zentrifugalpumpe gemäss Stand der Technik, teilweise im Schnitt,
- Fig. 2:: eine Schnittdarstellung der Pumpeneinheit der Zentrifugalpumpe aus Fig. 1,
- Fig. 3:: eine Schnittdarstellung eines ersten Ausführungsbeispiels einer erfindungsgemässen Pumpeneinheit,
- Fig. 4:: wie Fig. 3, jedoch in einer Explosionsdarstellung,
- Fig. 5:: das Detail I aus Fig. 4,
- Fig. 6:: eine Schnittdarstellung verschiedener Varianten für die Dichtungsrippe (n),
- Fig. 7.:: eine Schnittdarstellung einer Variante für die Dichtflächen,
- Fig. 8:: eine Schnittdarstellung einer Variante für das erste Ausführungsbeispiel,
- Fig. 9:: wie Fig. 8, jedoch in einer Explosionsdarstellung,
- Fig. 10:: eine Schnittdarstellung eines zweiten Ausführungsbeispiels einer erfindungsgemässen Pumpeneinheit,
- Fig. 11:: wie Fig. 10, jedoch in einer Explosionsdarstellung,
- Fig. 12:: eine Schnittdarstellung einer Variante für das zweite Ausführungsbeispiel,
- Fig. 13:: wie Fig. 12, jedoch in einer Explosionsdarstellung,
- Fig. 14:: eine Schnittdarstellung eines dritten Ausführungsbeispiels einer erfindungsgemässen Pumpeneinheit in einer Explosionsdarstellung,
- Fig. 15:: eine Schnittdarstellung eines vierten Ausführungsbeispiels einer erfindungsgemässen Pumpeneinheit in einer Explosionsdarstellung,
- Fig. 16:: eine Schnittdarstellung eines fünften Ausführungsbeispiels einer erfindungsgemässen Pumpeneinheit in einer Explosionsdarstellung,
- Fig. 17: eine Schnittdarstellung eines sechsten Ausführungsbeispiels einer erfindungsgemässen Pumpeneinheit,
- Fig. 18:: wie Fig. 17, jedoch in einer Explosionsdarstellung, und
- Fig. 19: eine schematische Schnittdarstellung eines Ausführungsbeispiels einer erfindungsgemässen Zentrifugalpumpe.

Wie bereits vorangehend erläutert, zeigt Fig. 1 eine Zentrifugalpumpe 200' mit einem berührungslos magnetisch gelagerten und berührungslos magnetisch angetriebenen Rotor 10', die aus dem Stand der Technik bekannt ist. Fig. 2 zeigt in einer Schnittdarstellung die Pumpeneinheit 1' dieser Zentrifugalpumpe 200'.

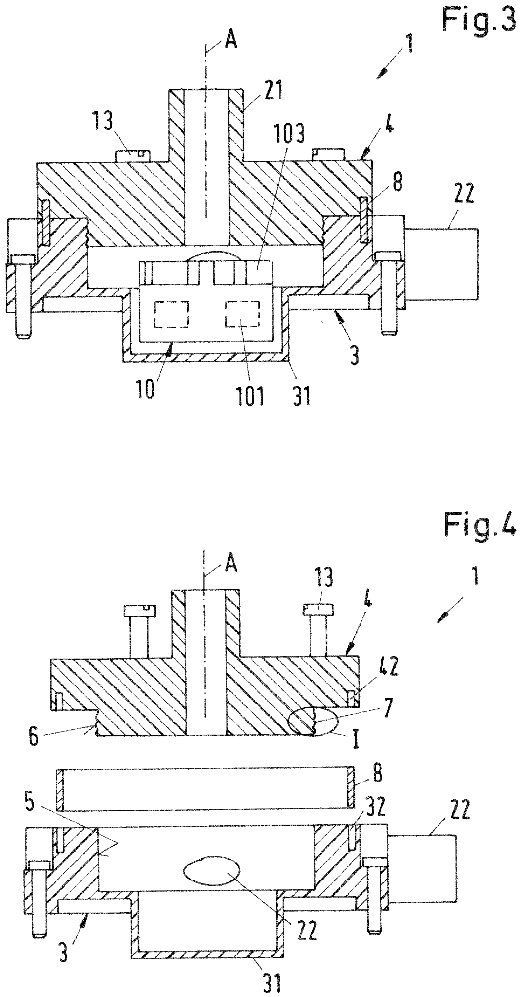

Fig. 3 zeigt in einer zu Fig. 2 analogen Darstellung ein erstes Ausführungsbeispiel einer erfindungsgemässen Pumpeneinheit, die gesamthaft mit dem Bezugszeichen 1 bezeichnet ist. Zum besseren Verständnis zeigt Fig. 4 das erste Ausführungsbeispiel der Pumpeneinheit 1 noch in einer Explosionsdarstellung.

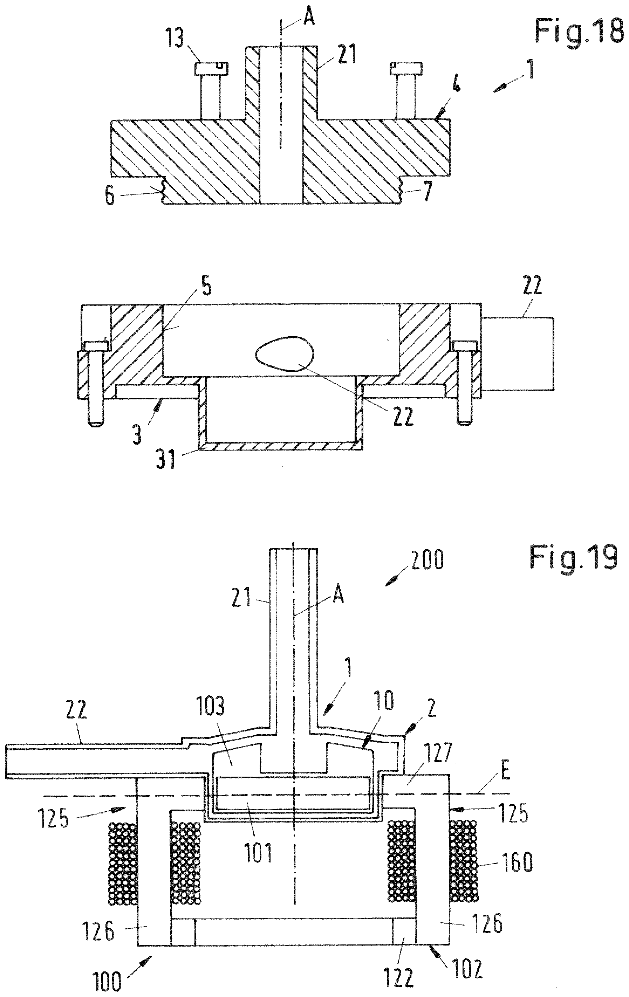

Die Pumpeneinheit 1 ist für eine Zentrifugalpumpe 200 (siehe Fig. 19) zum Fördern eines Fluids ausgestaltet und umfasst ein Pumpengehäuse 2 mit einem Einlass 21 und mit einem Auslass 22 für das Fluid. In dem Pumpengehäuse 2 ist ein Rotor 10 (in Fig. 4 nicht dargestellt) zum Fördern des Fluids angeordnet, welcher das Flügelrad bzw. das Laufrad der Pumpeneinheit 1 und damit der Zentrifugalpumpe 200 bildet. Der Rotor 10 ist um eine Solldrehachse rotierbar, welche eine axiale Richtung A definiert.

Eine zur axialen Richtung A senkrechte Richtung wird als radiale Richtung bezeichnet. Im Folgenden wird der Ausdruck "axial" mit der allgemein üblichen Bedeutung "in axialer Richtung" oder "bezüglich der axialen Richtung" verwendet. Der Begriff "radial" wird mit der allgemein üblichen Bedeutung "in radialer Richtung" oder "bezüglich der axialen Richtung" verwendet

Die Pumpeneinheit 1 ist für eine berührungslos magnetische Lagerung des Rotors 10 und für einen berührungslos magnetischen Antrieb des Rotors 10 ausgestaltet. Dies kann insbesondere in sinngemäss gleicher Weise realisiert sein, wie dies anhand der Fig. 1 und Fig. 2 erläutert ist. So kann die erfindungsgemässe Pumpeneinheit 1 bezüglich der magnetischen Lagerung und des magnetischen Antriebs in sinngemäss gleicher Weise ausgestaltet sein wie die Pumpeneinheit 1' in Fig. 2. Dazu umfasst der Rotor 10 der Pumpeneinheit 1 einen magnetisch wirksamen Kern 101, der beispielsweise als permanentmagnetische Ring oder permanentmagnetische Scheibe ausgestaltet ist, und von einer Kunststoffummantelung umschlossen wird.

Der Rotor 10 umfasst ferner eine Mehrzahl von Flügeln 103, um das Fluid vom Einlass 21 zum Auslass 22 zu fördern. Die Flügel 103 sind auf der Kunststoffummantelung des magnetisch wirksamen Kerns angeordnet. Die Flügel 103 bestehen vorzugsweise aus Kunststoff und können beispielsweise einstückig mit der Kunststoffummantelung ausgestaltet sein. Natürlich ist es auch möglich, die individuellen Flügel 103 oder die Gesamtheit der Flügel 103 in einem separaten Fertigungsprozess herzustellen und sie dann mit der Kunststoffummantelung des magnetisch wirksamen Kerns 101 zu verbinden, beispielsweise mittels eines Schweissprozesses.

Das von dem Rotor 10 gebildete Laufrad mit den Flügeln 103 ist vorzugsweise als radiales Laufrad ausgestaltet, welches von dem Fluid vom Einlass 21 in axialer Richtung A angeströmt wird, und das Fluid dann in eine radiale Richtung umlenkt.

Das Pumpengehäuse 2 umfasst ein Bodenteil 3 und einen Deckel 4 zum Verschliessen des Bodenteils 3, wobei das Bodenteil 3 einen zylindrischen Becher 31 zur Aufnahme des Rotors 10 aufweist. Der Becher 31 ist vorzugsweise so ausgestaltet und angeordnet, dass er in eine Ausnehmung einer Antriebseinheit 100 (Fig. 19) einsetzbar ist. Dies kann insbesondere in analoger Weise realisiert sein, wie dies anhand der Fig. 1 erläutert wurde. Der Becher ist dann also derart angeordnet und ausgestaltet, dass er in die Ausnehmung 121' (Fig. 1) im Motorgehäuse 120' der Antriebseinheit 100` einsetzbar ist, und der magnetisch wirksame Kern 101 zwischen den Querschenkeln 127` der Spulenkerne 125' angeordnet ist.

Ein wesentlicher Aspekt für die Betriebssicherheit der Pumpeneinheit 1 ist eine zuverlässig dichtende Verbindung zwischen dem Bodenteil 3 und dem Deckel 4, sodass Leckagen des Fluids aus dem Inneren des Pumpengehäuses 2 zwischen dem Bodenteil 3 und dem Deckel 4 hindurch in den Aussenraum ausserhalb des Pumpengehäuses zuverlässig verhindert werden können. Diese dichtende Verbindung muss auch bei hohen Temperaturen von beispielsweise bis zu 220°C und/oder bei hohen Drücken und/oder für chemisch sehr aggressive Fluide, wie beispielsweise Schwefelsäure gewährleistet sein.

Erfindungsgemäss weist dazu das Bodenteil 3 eine im Wesentlichen ringförmige erste Dichtfläche 5 zum dichtenden Zusammenwirken mit dem Deckel 4 auf, und der Deckel 4 eine im Wesentlichen ringförmige zweite Dichtfläche 6, die zum dichtenden Zusammenwirken mit der ersten Dichtfläche 5 ausgestaltet ist. Dazu ist eine der beiden Dichtflächen 5 oder 6 als Rippenfläche mit mindestens einer radialen Dichtungsrippe 7 ausgestaltet, die sich in Umfangsrichtung entlang der gesamten Dichtfläche 5 oder 6 erstreckt, während die andere der beiden Dichtflächen 6 oder 5 als glatte Fläche ausgestaltet ist.

Bei dem in Fig. 3 und Fig. 4 dargestellten Ausführungsbeispiel ist die erste Dichtfläche 5, also die Dichtfläche 5 des Bodenteils 3, als die glatte Fläche ausgestaltet, und die zweite Dichtfläche 6, also die Dichtfläche 6 des Deckels 4, als die Rippenfläche.

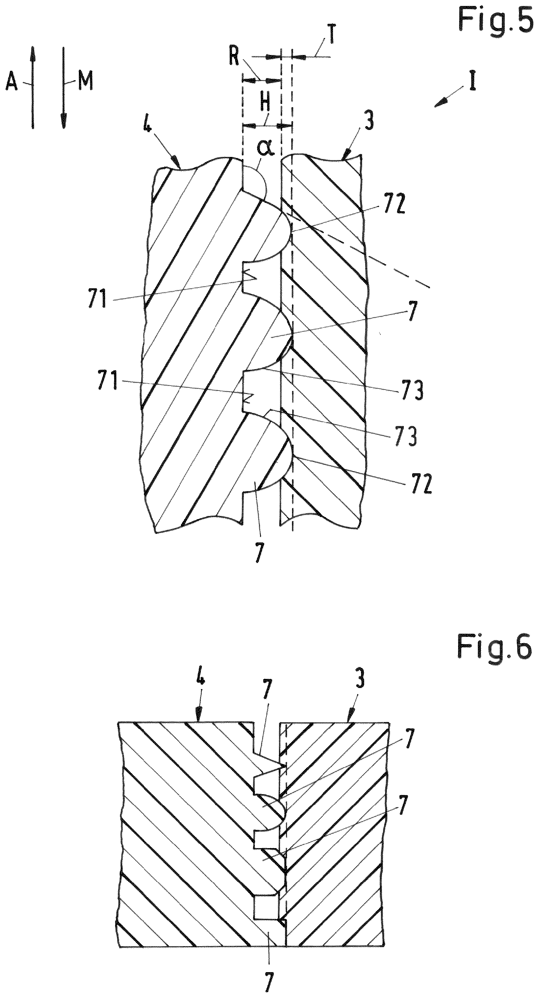

Zum besseren Verständnis zeigt Fig. 5 noch eine vergrösserte Darstellung des Details I aus Fig. 4, sodass das dichtende Zusammenwirken der ersten Dichtfläche 5 und der zweiten Dichtfläche 6 besser zu erkennen ist.

Vorzugsweise umfasst die Rippenfläche mehrere - beim ersten Ausführungsbeispiel genau drei - radiale Dichtungsrippen 7, von denen sich jede vollständig entlang der gesamten Rippenfläche erstreckt, wobei die individuellen Dichtungsrippen 7 bezüglich der axialen Richtung A benachbart zueinander angeordnet sind. Jede Dichtungsrippe 7 ist als geschlossener kreisförmiger Ring ausgestaltet. Jede radiale Dichtungsrippe 7 ist so ausgestaltet, dass sie radiale Kräfte aufnehmen kann. Hierzu ist es zwar bevorzugt, aber nicht notwendig, dass die Dichtungsrippe 7 senkrecht bzw. rechtwinklig zur axialen Richtung A ausgerichtet ist. Es sind auch Ausgestaltungen möglich, bei denen die Dichtungsrippe 7 schräg, also unter einem von 90° verschiedenen Winkel zur axialen Richtung A auf der Dichtfläche 5 oder 6 angeordnet ist. Wesentlich ist nur, dass die radiale Dichtungsrippe 7 in radialer Richtung eine ausreichende Erstreckung hat, um radiale Kräfte aufnehmen zu können.

Es versteht sich, dass die Anzahl von drei Dichtungsrippen 7 beispielhaft zu verstehen ist. Es können auch mehr als drei oder weniger als drei Dichtungsrippen 7 in der als Rippenfläche ausgestalteten Dichtfläche 5 oder 6 vorgesehen sein.

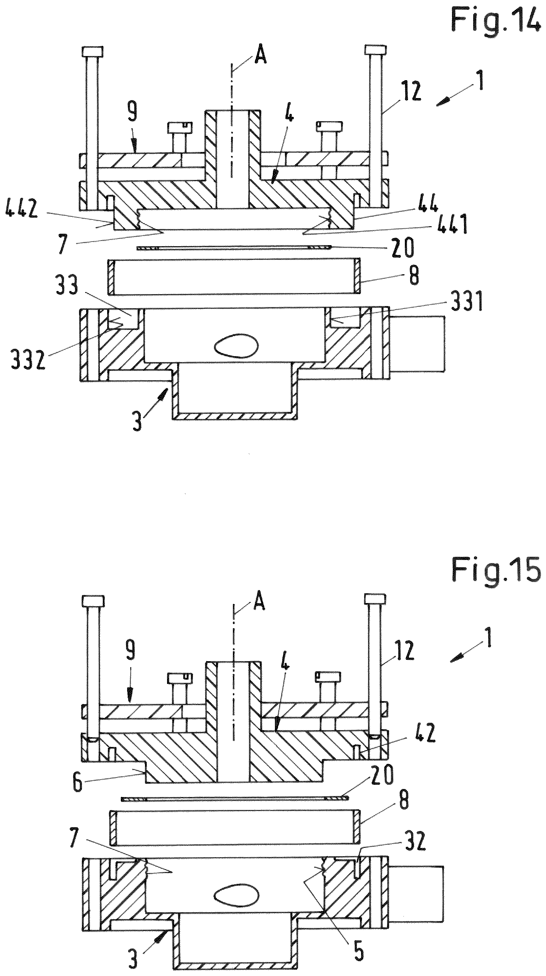

Sowohl bei dem ersten Ausführungsbeispiel des erfindungsgemässen Pumpengehäuses 1 als auch bei allen weiteren im Folgenden noch beschriebenen Ausführungsbeispielen sind Varianten möglich, bei denen die erste Dichtfläche 5 als Rippenfläche ausgestaltet ist, und die zweite Dichtfläche 6 als glatte Fläche. Es sind also bei allen Ausführungsbeispielen immer beide Ausführungsformen möglich, nämlich, dass jede Dichtungsrippe 7 am Bodenteil 3 vorgesehen ist und die zweite Dichtfläche am Deckel 6 als glatte Fläche ausgestaltet ist, und, dass jede Dichtungsrippe 7 am Deckel 4 vorgesehen ist und die erste Dichtfläche 5 am Bodenteil 3 als glatte Fläche ausgestaltet ist. Mit beispielhaftem Charakter zeigt Fig. 15 eine Ausführungsform, bei welcher die Dichtrippen 7 in der ersten Dichtfläche 5 angeordnet sind, also in der Dichtfläche 5 des Bodenteils 3.

Mit dem Begriff, dass eine der Dichtflächen 5, 6 als "glatte Fläche" ausgestaltet ist, ist gemeint, dass dies Fläche keine Vertiefungen oder Ausnehmungen, wie beispielsweise Nuten aufweist, in welche die Dichtungsrippen 7 eingreifen könnten. Natürlich ist es möglich, dass die glatte Fläche durch die Dichtungsrippen 7 plastisch oder elastisch deformiert wird (siehe z.B. Fig. 5), aber in der als glatte Fläche ausgestalteten Dichtfläche 5 oder 6 ist keine Textur bzw. keine Struktur vorgesehen, in welche die Dichtungsrippen 7 eingreifen könnten, also insbesondere keine Nuten. Die Dichtwirkung zwischen der als Rippenfläche ausgestalteten Dichtfläche 5 oder 6 und der als glatte Fläche ausgestalteten Dichtfläche 6 oder 5 basiert auf der Pressung der Dichtungsrippen 7 gegen die glatte Fläche und nicht auf einem Eingreifen der Dichtungsrippen 7 in Nuten oder sonstige Ausnehmungen.

Das Pumpengehäuse 2 ist vorzugsweise aus einem Kunststoff gefertigt. Bevorzugte Kunststoffe sind beispielsweise Perfluoralkoxy-Polymere (PFA) oder Polytetrafluorethylen (PTFE), weil dies eine besonders hohe chemische Resistenz aufweisen und auch durch chemisch sehr aggressive Substanzen, beispielsweise Schwefelsäure nicht angegriffen bzw. zersetzt werden. Zudem sind PFA und PTFE besonders reine Kunststoffe, in denen keine Zusatzstoffe vorgesehen sind, und deren Molekülkomplexe chemisch sehr stabil, also resistent gegen aggressive Substanzen sind.

Der Auslass 22 für das Fluid ist vorzugsweise im Bodenteil 3 des Pumpengehäuses 2 angeordnet. Insbesondere ist der Auslass 22 bezüglich der axialen Richtung A zwischen dem Becher 31 und der ersten Dichtfläche 5 angeordnet. Das bedeutet, dass der Auslass 22 darstellungsgemäss (z. B. Fig. 3, Fig.4) unterhalb aller Dichtungsrippen 7 angeordnet ist, und zwar unabhängig davon, ob die Dichtungsrippen 7 in der zweiten Dichtfläche 6 (z.B. Fig. 4) oder in der ersten Dichtfläche (Fig. 15) angeordnet sind.

Die Dichtungsrippen 7 sind vorzugsweise integraler Bestandteil der ersten oder der zweiten Dichtfläche 5, 6. Die Dichtungsrippen 7 können beispielsweis in einem Spritzverfahren hergestellt werden. Wenn beispielsweise der Deckel 4 und das Bodenteil 3 in einem Spritzgiessverfahren hergestellt werden, so können die Dichtungsrippen 7 im Rahmen dieses Spritzgiessens durch eine dementsprechende Ausgestaltung der Spritzgiessform bzw. des Werkzeugs erzeugt werden. Es ist aber auch möglich, die Dichtungsrippen 7 in einem subtraktiven Bearbeitungsverfahren herzustellen. Die Dichtungsrippen 7 können beispielsweise mittels einer spanabhebenden Bearbeitung, z.B. Fräsen, aus der ersten oder der zweiten Dichtfläche 5, 6 herausgearbeitet werden.

Das dichtende Zusammenwirken zwischen der ersten Dichtfläche 5 und der zweiten Dichtfläche 6 basiert auf einem Presssitz zwischen dem Deckel 4 und dem Bodenteil 3, was anhand von Fig. 5 noch näher erläutert wird.

Gemäss einer bevorzugten Ausgestaltung umfasst die Pumpeneinheit 1 ferner ein radiales Verstärkungselement 8, welches ringförmig ausgestaltet ist, und sich radial aussenliegend um die beiden Dichtflächen 5, 6 herum erstreckt. Vorzugsweise ist das radiale Verstärkungselement 8 als ein metallischer Ring ausgestaltet, der mit einer Kunststoffbeschichtung vollständig umschlossen ist. Für den metallischen Ring ist ein rostfreier Stahl bzw. ein Edelstahl bevorzugt. Für die Kunststoffbeschichtung ist ein chemisch hoch resistenter Kunststoff bevorzugt. Beispiele für solche bevorzugten Kunststoffe sind PTFE, PFA, ECTFE (Ethylenchlortrifluorethylen), PP (Polypropylen), ETFE (Ethylen-Tetrafluorethylen), PE (Polyethylen). Alternativ ist es auch möglich, das radiale Verstärkungselement 8 vollständig aus einem starken bzw. stabilen Kunststoff herzustellen.

Das radiale Verstärkungselement 8 stabilisiert die beiden Dichtflächen 5, 6, sodass diese auch bei höheren Drücken im Pumpengehäuse 2 noch besser in dichtendem Kontakt miteinander bleiben. Das radiale Verstärkungselement 8 trägt dazu bei, Relativbewegungen zwischen den beiden Dichtflächen zu vermeiden, die schlimmstenfalls zum Öffnen von Spalten führen könnten, durch welche das Fluid aus dem Pumpengehäuse 2 austreten könnte. Eine weitere Funktion des radialen Verstärkungselements 8 ist es, einem Kriechen des Bodenteils 3 oder des Deckels 4 entgegenzuwirken. Ferner kann das radiale Verstärkungselement 8 auch so ausgestaltet werden, dass es eine Federwirkung in radialer Richtung nach ihnen ausübt, welche den Presssitz zwischen den Dichtflächen 5, 6 beaufschlagt. Hierdurch ist es möglich, falls sich beispielsweise das Bodenteil 3 oder der Deckel 4 verziehen, den Presssitz zwischen dem Deckel 4 und dem Bodenteil 3 nachzuführen.

Vorzugsweise ist das radiale Verstärkungselement 8 so angeordnet, dass es sowohl in den Deckel 4 als auch in das Bodenteil 3 eingreift, sodass das radiale Verstärkungselement 8 bezüglich der axialen Richtung A die Grenzfläche übergreift, an welcher das Bodenteil 3 und der Deckel 4 aneinander anliegen. Dazu ist in dem Deckel 4 eine Umfangsnut 42 (Fig. 4) vorgesehen, und in dem Bodenteil 3 ist eine Umfangsnut 32 vorgesehen, wobei die Umfangsnuten 32, 42 so angeordnet sind, dass sie miteinander fluchten, sodass sie im zusammengesetzten Zustand des Pumpengehäuses 2 (Fig. 3) eine Kavität für das radiale Verstärkungselement 8 bilden, welche das radiale Verstärkungselement 8 umschliesst.

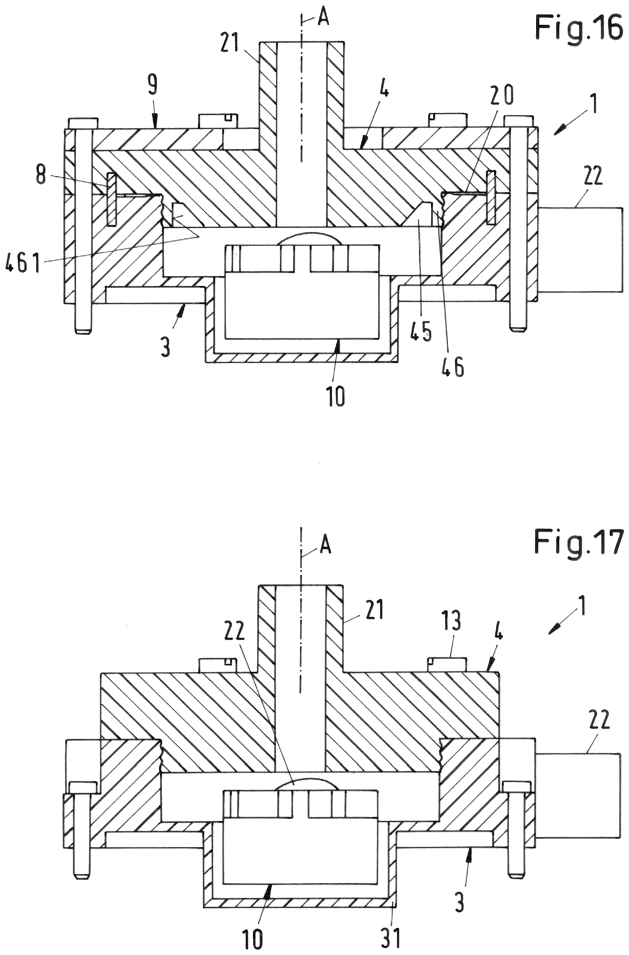

Es sind jedoch auch Ausgestaltungen ohne das radiale Verstärkungselement 8 möglich (siehe z. B. Fig. 17, Fig. 18).

Der Presssitz zwischen dem Deckel 4 und dem Bodenteil 3 ist in der vergrösserten Darstellung des Details I in Fig. 5 dargestellt. Wie bereits erwähnt, ist jede radiale Dichtungsrippe 7 kreisringförmig ausgestaltet, wobei die individuellen Dichtungsrippen 7 bezüglich der axialen Richtung A benachbart zueinander angeordnet sind. Zwischen zwei benachbarten Dichtungsrippen 7 ist jeweils ein Tal 71 vorgesehen, wobei jedes Tal 71 von der als glatten Fläche ausgestalteten Dichtfläche - hier die erste Dichtfläche 5 - einen radialen Abstand R hat.

Jede Dichtungsrippe 7 hat einen Berg 72, womit diejenige Stelle der Dichtungsrippe 7 gemeint ist, welche in radialer Richtung gemessen am weitesten von dem benachbarten Tal 71 entfernt ist. Jede Dichtungsrippe 7 hat eine Höhe H, womit der in radialer Richtung gemessene senkrechte Abstand zwischen dem Berg 72 und dem benachbarten Tal 71 bezeichnet wird. Der Berg 72 ist über jeweils eine Wand 73 mit den benachbarten Tälern verbunden.

Die Höhe H der Dichtungsrippe bezieht sich dabei auf den Zustand, wenn der Deckel 4 noch nicht mit dem Bodenteil 3 zusammengesetzt ist, also auf den Zustand wie er beispielsweise in Fig. 4 dargestellt ist. Nachdem der Deckel mit dem Bodenteil zusammengesetzt ist, also in dem Zustand, wie er beispielsweise in Fig. 3 dargestellt ist, tauchen die Dichtungsrippen 7, beispielsweise durch Deformation der als glatten Fläche ausgestalteten ersten Dichtfläche 5, um eine Eintauchtiefe T in die glatte Fläche ein. Die Eintauchtiefe T gibt also die in radialer Richtung gemessene Differenz zwischen der Position des Bergs 72 und dem nicht deformierten Bereich der glatten Oberfläche an, welcher einem der Täler 71 gegenüberliegt.

Um das Pumpengehäuse 2 aus dem Bodenteil 3 und dem Deckel 4 zusammenzusetzen, wird vorzugsweise der Deckel 4 in einer Einbaurichtung M mit dem Bodenteil 3 zusammengeführt, bis die erste Dichtfläche 5 dichtend mit der zweiten Dichtfläche 6 zusammenwirkt. Dieser Zustand ist erreicht, wenn der Deckel 4 auf dem Bodenteil 3 aufliegt. Die Einbaurichtung M ist hier die axiale Richtung A. Nachdem der Deckel 4 über den Presssitz mit dem Bodenteil 3 verbunden ist, wird der Deckel 4 am Bodenteil 3 fixiert, beispielsweise mittels mehrerer Befestigungsschrauben 13, die in axialer Richtung A durch den Deckel 4 hindurchgreifen und in das Bodenteil 3 eingreifen.

Dadurch, dass die Dichtungsrippen 7 als radiale Dichtungsrippen 7 mit der Höhe H ausgestaltet sind und die Einbaurichtung M die axiale Richtung A ist, erstrecken sich die Dichtungsrippen 7 quer, beispielsweise senkrecht, zur Einbaurichtung M. Dies ist eine vorteilhaftere Ausgestaltung als beispielsweise ein Dichtelement welches sich in der Einbaurichtung erstreckt, also beispielsweise ein axiales Dichtungselement.

Die Stärke des Presssitzes zwischen dem Deckel 4 und dem Bodenteil 3 hängt unter anderem von dem radialen Abstand R, der Höhe H und der Eintauchtiefe T ab, wobei insbesondere die Eintauchtiefe T von den Materialeigenschaften des Materials oder der Materialien abhängt, aus denen der Deckel 4 und das Bodenteil 3 gefertigt sind.

In der Praxis hat es sich bewährt, dass die Höhe H mindestens so gross, vorzugsweise grösser ist als der radiale Abstand R. Die Eintauchtiefe T kann - zumindest näherungsweise - null betragen, sodass die Dichtungsrippe 7 an der glatten Fläche anliegt. Bevorzugt ist es jedoch, wenn die Eintauchtiefe T grösser als null ist, sodass die Dichtungsrippe 7 in die glatte Fläche - hier die erste Dichtfläche 5 eintaucht. Der radiale Abstand R kann - zumindest näherungsweise - null betragen. Bevorzugt ist es jedoch, wenn der radiale Abstand R grösser als null ist, also der am Tal 71 gemessene Durchmesser des Deckels 4 kleiner als der Innendurchmesser der glatten Fläche, hier also der ersten Dichtfläche 5.

Der radiale Abstand R ist vorzugsweise nicht kleiner als null. Falls der radiale Abstand R kleiner als null ist, ist der am Tal 71 gemessene Durchmesser des Deckels 4 grösser als der Innendurchmesser der glatten Fläche, hier also der ersten Dichtfläche 5. Falls der radiale Abstand kleiner als null ist, so wirkt sich das negativ auf die Trennbarkeit von Deckel 4 und Bodenteil 3 aus. Eine solche Trennung kann beispielsweise notwendig sein, weil der Rotor 10 gewechselt werden muss.

Insbesondere für die Herstellung des Deckels 4 mit den Dichtungsrippen 7 (oder natürlich alternativ auch für die Herstellung des Bodenteils 3 mit den Dichtungsrippen 7) ist es vorteilhaft, wenn der Verbindungswinkel α zwischen dem Tal 71 und der Wand 73, jeweils mindestens 90° und vorzugsweise grösser als 90° ist, sodass im Übergangsbereich zwischen der Wand 73 und dem Tal 71 jeweils ein Hinterschnitt vermieden wird. Dies ist insbesondere vorteilhaft, wenn die Dichtungsrippen in einem Spritzgiessverfahren hergestellt werden.

Bezüglich des Profils der Dichtungsrippen 7, womit ihr in Fig. 5 gezeigter Querschnitt in einem Schnitt in axialer Richtung A gemeint ist, sind gerundete Profile bevorzugt, weil diese das Zusammenfügen bzw. das Trennen von dem Deckel 4 und dem Bodenteil 3 erleichtern. Ferner sind solche Profile bevorzugt, bei denen die beiden Wände 73 der Dichtungsrippe 7, die ihren Berg 72 mit den beiden benachbarten Tälern 71 verbinden, symmetrisch bezüglich des Berges 72 ausgestaltet sind.

Fig. 6 zeigt mit beispielhaftem Charakter vier verschiedene Varianten für die Ausgestaltung des Profils der Dichtungsrippen 7 in einem Schnitt in axialer Richtung A. Die darstellungsgemäss oberste Dichtungsrippe 7 hat ein im Wesentlichen dreieckiges Profil. Die darstellungsgemäss zweite Dichtungsrippe 7 von oben hat an ihrem radial innenliegenden Ende, also dort, wo die Täler 71 angeordnet sind, ein rechteckiges Profil. Das radial aussenliegende Ende der Dichtungsrippe 7 ist mit einem halbkreisförmigen Profil ausgestaltet, welches sich in radialer Richtung an das rechteckige Profil anschliesst. Die darstellungsgemäss dritte Dichtungsrippe 7 von oben hat ein im Wesentlichen rechteckiges Profil, wobei die Ecken, welche der ersten Dichtfläche 5 zugewandt sind, jeweils mit einer Fase versehen sind. Die darstellungsgemäss unterste Dichtungsrippe hat ein rechteckiges Profil ohne Fase.

Es versteht sich, dass noch zahlreiche andere Profile für die Dichtungsrippen 7 möglich sind. Es sind Ausgestaltungen möglich bei denen auf der Dichtfläche 5 oder 6 alle Dichtungsrippen 7 das gleiche Profil haben. Ferner sind Ausgestaltungen möglich, bei denen die Dichtungsrippen 7 auf der Dichtfläche 5 oder 6 mindestens zwei unterschiedliche Profile aufweisen.

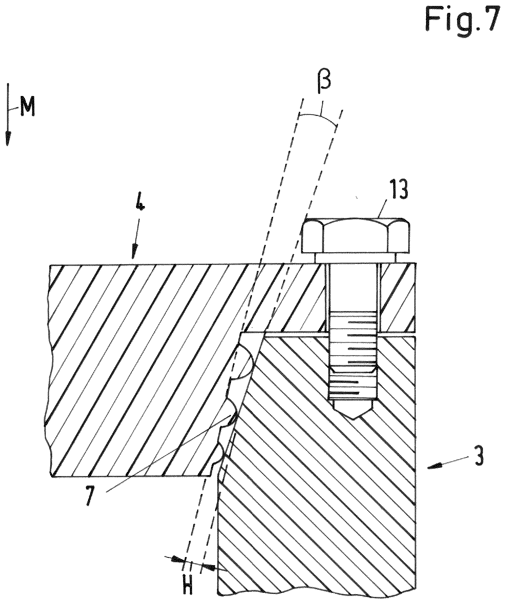

Fig. 7 zeigt eine Variante für die Ausgestaltung der Dichtflächen 5 und 6. Bei dieser Variante ist die als glatte Fläche ausgestaltete Dichtfläche 5 oder 6, hier mit beispielhaftem Charakter die erst Dichtfläche 5, kegelstumpfförmig, das heisst als Mantelfläche eines Kegelstumpfes, ausgestaltet. Der Flächennormalenvektor der ersten Dichtfläche 5 ist also nicht mehr senkrecht zur axialen Richtung orientiert, sondern schliesst mit der axialen Richtung A einen von 90° verschiedenen Winkel ein. Der Kegelstumpf ist dabei so ausgerichtet, dass er sich in der Einbaurichtung M verjüngt. Das heisst, der von der Dichtfläche 5 umschlossene Durchmesser senkrecht zur axialen Richtung A nimmt in Richtung auf den Rotor 10 gesehen ab. Die erste Dichtfläche 5 bildet also quasi einen Trichter, der sich verjüngt, wenn man vom Deckel 4 in Richtung des Rotors 10 schaut.

Vorzugsweise, aber nicht notwendigerweise, ist auch die zweite Dichtfläche 6, die hier als Rippenfläche mit den Dichtungsrippen 7 ausgestaltet ist, kegelstumpfförmig ausgestaltet. Dabei ist die zweite Dichtfläche 6 als Mantelfläche eines Kegelstumpfes ausgestaltet, der spitzwinkliger ist als der Kegelstumpf, dessen Mantelfläche die erste Dichtfläche 5 bildet. Durch diese Ausgestaltung schliessen die erste Dichtfläche 5 und die zweite Dichtfläche 6 einen Spaltwinkel β ein, der von null verschieden ist. Wie in Fig. 7 dargestellt ist der Spaltwinkel β der Winkel zwischen der als glatte Fläche ausgestalteten ersten Dichtfläche 5 und einer Gerade, die durch alle Täler 71 der zweiten Dichtfläche 6 verläuft. Damit die radiale Dichtwirkung in ausreichender Weise erhalten bleibt, ist der Spaltwinkel β höchstens 60° und vorzugsweise kleiner als 30°.

Auch ist es bevorzugt, aber nicht notwendig, wenn die Dichtungsrippen 7 - wie in Fig. 7 dargestellt unterschiedliche Höhen H haben. Die jeweilige Höhe H der Dichtungsrippen 7 nimmt ab, je mehr sich der von der Rippenfläche - hier die zweite Dichtfläche 6 - umschlossene Raum verjüngt. Gemäss der Darstellung in Fig. 7 hat die oberste Dichtungsrippe 7 die grösste Höhe H, und die darstellungsgemäss unterste Dichtungsrippe 7 hat die kleinste Höhe H.

Ein Vorteil dieser konischen Ausgestaltung der Dichtflächen 5 und 6 ist es, dass der Presssitz, also insbesondere sie Stärke des Presssitzes, durch die Befestigungsschrauben 13 eingestellt werden kann. Je weiter man durch Drehen der Befestigungsschrauben 13 den von der zweiten Dichtfläche 6 umschlossenen Konus in der Einbaurichtung M in den von der ersten Dichtfläche 5 umschlossenen Konus hineinschiebt, um so stärker wird der Presssitz zwischen dem Deckel 4 und dem Bodenteil 3.

Ferner hat diese Ausgestaltung den Vorteil, dass der Presssitz nachgezogen werden kann, beispielsweise bei einem Langzeitkriechen des Deckels 4 und/oder des Bodenteils 3.

Zudem ermöglicht die konische Ausgestaltung der Dichtflächen 5, 6 eine einfachere Trennung des Deckels 4 von dem Bodenteil 3. Die Gefahr von Beschädigungen beim Trennen des Deckels 4 von dem Bodenteil 3 entgegen der Einbaurichtung M wird deutlich reduziert.

Es sind auch noch andere Geometrien der im Wesentlichen ringförmigen Dichtflächen 5, 6 möglich, so können die beiden Dichtflächen 5, 6 beispielsweise so ausgestaltet sein, dass sie ein Ellipsoid umschliessen, oder derart, dass sie eine Spirale umschliessen. Letzteres ist bevorzugt für solche Ausgestaltungen, bei welchen das Pumpengehäuse 2 als Spiralgehäuse (Volute) ausgestaltet ist.

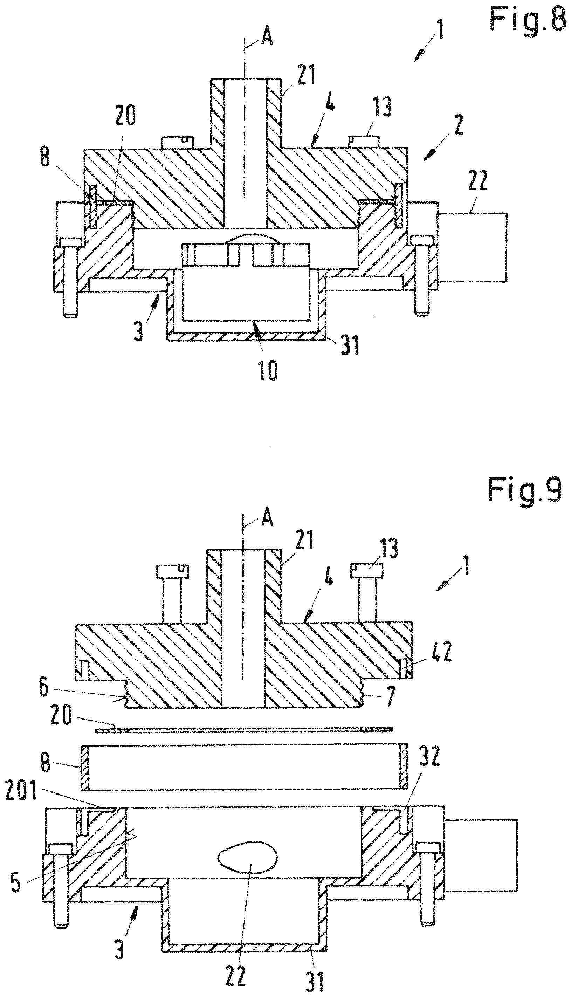

Fig. 8 zeigt in einer zu Fig. 3 analogen Schnittdarstellung eine Variante des ersten Ausführungsbeispiels der erfindungsgemässen Pumpeneinheit 1. Zum besseren Verständnis zeigt Fig. 9 diese Variante noch in einer Explosionsdarstellung.

Bei der in Fig. 8 und Fig. 9 dargestellten Variante ist zusätzlich eine ringförmige Sicherheitsdichtung 20 vorgesehen, welche im Fehlerfall ein Austreten des Fluids zwischen dem Bodenteil 3 und dem Deckel 4 verhindert. Bezüglich der axialen Richtung A ist die Sicherheitsdichtung 20 in der Kontaktfläche zwischen dem Bodenteil 3 und dem Deckel 4 angeordnet. Bezüglich der radialen Richtung ist die Sicherheitsdichtung 20 zwischen den beiden Dichtflächen 5, 6 einerseits und dem radialen Verstärkungselement 8 angeordnet, das heisst, die Sicherheitsdichtung 20 ist radial aussenliegend bezüglich der ersten Dichtfläche 5 und der zweiten Dichtfläche 6 angeordnet. Im fehlerfreien Betriebszustand der Pumpeneinheit 1 kommt die Sicherheitsdichtung 20 folglich nicht mit dem Fluid in Kontakt. Durch das dichtende Zusammenwirken der ersten Dichtungsfläche 5 mit der zweiten Dichtfläche 6 kann das Fluid nicht bis an die Sicherheitsdichtung 20 vordringen, sodass auch umgekehrt keine Gefahr besteht, dass das Fluid durch die Sicherheitsdichtung 20 verunreinigt wird.

Sollte es im Betrieb jedoch zu einer Störung kommen, durch welche die Dichtwirkung zwischen den beiden Dichtflächen 5, 6 nicht mehr im ausreichenden Masse gewährleistet ist, so verhindert die Sicherheitsdichtung 20 einerseits, dass das Fluid ungewollt bzw. unkontrolliert aus dem Pumpengehäuse 2 austritt, sodass beispielsweise aggressive oder anderweitig gefährlich Fluide nicht in die Umwelt bzw. in den Aussenraum des Pumpengehäuses 2 gelangen können. Andererseits verhindert die Sicherheitsdichtung 20 in solchen Fehlerfällen, dass von aussen Substanzen in den Innenraum des Pumpengehäuses eindringen, welche zur Kontamination des Fluids und damit zur Unbrauchbarkeit des Fluids oder der mit dem Fluid behandelten Produkte, beispielsweise Wafer in der Halbleiterindustrie, führen können.

Solche Fehlerfälle, die zu einer nicht mehr ausreichenden Dichtwirkung durch die beiden Dichtflächen 5, 6 führen können, basieren beispielsweise auf Kriecheffekten, insbesondere Langzeitkriecheffekten, oder auf druck- und/oder temperaturinduzierten Deformationen, beispielsweise des Deckels 4 oder des Bodenteils 3.

Die Sicherheitsdichtung 20 ist vorzugsweise als eine ringförmige Flachdichtung ausgestaltet.

Die Sicherheitsdichtung 20 besteht vorzugsweise aus einem Kunststoff, beispielsweise aus einem Kunststoff, der üblicherweise zum Abdichten bei hohen Temperaturen und/oder chemisch aggressiven Fluiden verwendet wird. Die Sicherheitsdichtung 20 kann beispielsweise aus dem gleichen Kunststoff bestehen wie das Pumpengehäuse 2. Beispielsweise kann die Sicherheitsdichtung 20 aus PTFE bestehen. Hierbei ist es bevorzugt, dass die Sicherheitsdichtung 20 aus ePTFE (expanded PTFE) besteht, insbesondere weil ePTFE bessere elastische Eigenschaften aufweist als PTFE.

Natürlich ist es auch möglich, an sich bekannte Elastomere für die Sicherheitsdichtung 20 zu verwenden. Ferner ist es möglich, dass die Sicherheitsdichtung als radialer oder axialer O-Ring ausgestaltet ist.

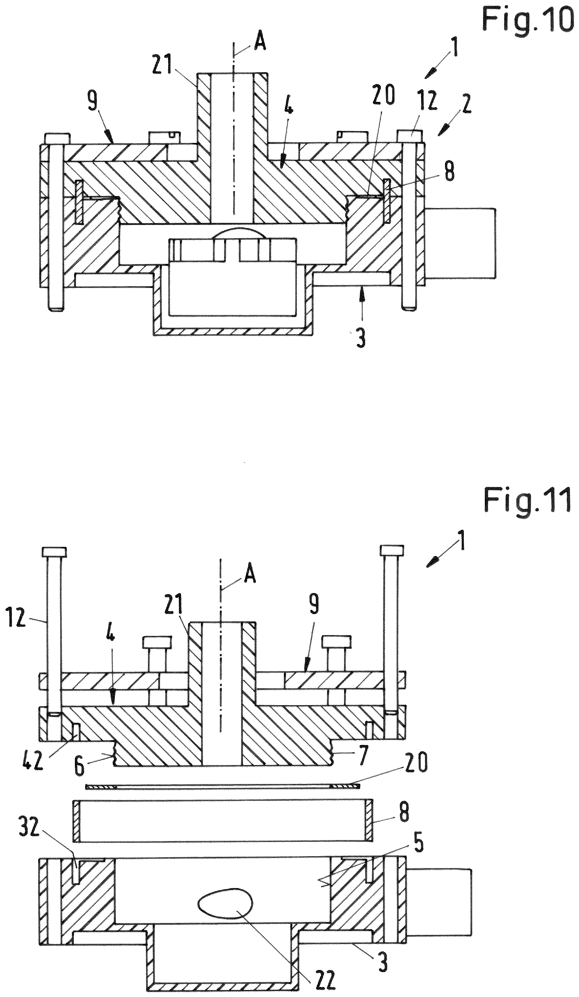

Fig. 10 zeigt in einer zu Fig. 3 analogen Darstellung eine Schnittdarstellung eines zweiten Ausführungsbeispiels einer erfindungsgemässen Pumpeneinheit 1. Zum besseren Verständnis zeigt Fig. 11 das zweite Ausführungsbeispiel noch in einer Explosionsdarstellung.

Im Folgenden wird nur auf die Unterschiede zu dem ersten Ausführungsbeispiel eingegangen. Gleiche Teile oder von der Funktion her gleichwertige Teile des zweiten

Ausführungsbeispiels sind mit den gleichen Bezugszeichen bezeichnet wie bei dem ersten Ausführungsbeispiel. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen des ersten Ausführungsbeispiels und der Varianten in gleicher Weise oder in sinngemäss gleicher Weise auch für das zweite Ausführungsbeispiel gelten.

Bei dem zweiten Ausführungsbeispiel der Pumpeneinheit 1 ist zusätzlich auf dem Deckel 4 ein axiales Verstärkungselement 9 vorgesehen, welches in axialer Richtung A wirkende Kräfte aufnehmen kann. Das axiale Verstärkungselement 9 ist hier als ringscheibenförmige Platte ausgestaltet, welche den Einlass 21 umgibt und auf dem Deckel 4 aufliegt, sodass der Deckel 4 zwischen dem axialen Verstärkungselement 9 und dem Bodenteil 3 angeordnet ist.

Ferner ist eine Mehrzahl von Spannschrauben 12 vorgesehen, von denen sich jede in axialer Richtung A vollständig durch das axiale Verstärkungselement 9 und das Pumpengehäuse 2 erstreckt, sodass jede Spannschraube 12 bezüglich der axialen Richtung A darstellungsgemäss unten aus dem Pumpengehäuse 2 herausragt. Die Spannschrauben 12 sind für das Eingreifen in die Antriebseinheit 100 (Fig. 19) ausgestaltet, wenn die Pumpeneinheit 1 mit der Antriebseinheit 100 zu der Zentrifugalpumpe 200 zusammengesetzt wird. Vorzugsweise sind dazu in dem Motorengehäuse der Antriebseinheit 100, das beispielsweise wie das Motorengehäuse 120' in Fig. 1 ausgestaltet sein kann, Gewinde vorgesehen, in welche die Spannschrauben 12 eingreifen.

Die Spannschrauben 12 sind radial aussenliegend bezüglich des radialen Verstärkungselement 8 angeordnet. Mittels der Spannschrauben 12 kann das Pumpengehäuse 2 zwischen der Antriebseinheit 100 und dem axialen Verstärkungselement 9 eingespannt werden, wodurch sich die Stabilität des Pumpengehäuses bezüglich der axialen Richtung A deutlich erhöhen lässt. Eine solche erhöhte Stabilität ist insbesondere vorteilhaft bei Anwendungen, bei denen mit der Pumpeneinheit 1 sehr hohe Drücke erzeugt werden, oder wenn Fluide bei sehr hohen Temperaturen von beispielsweise 200°C oder mehr gefördert werden, oder wenn die Pumpeneinheit 1 von ihren Abmessungen sehr gross ausgestaltet ist. Durch das axiale Verstärkungselement kann einem Kriechen, das durch axiale Kräfte verursacht wird, noch effektiver entgegengewirkt werden.

Als Variante ist es auch möglich, das axiale Verstärkungselement 9 mit einer Federwirkung auszugestalten, beispielsweise mittels Federscheiben, sodass das axiale Verstärkungselement 9 eine Federkraft ausübt, welche vorteilhaft ist, um ein Kriechen von Komponenten in axialer Richtung A noch weiter zu reduzieren. Hierdurch ist auch ein Nachstellen der Dichtkräfte möglich, über welche die beiden Dichtflächen 5, 6 zusammenwirken.

Die Bezeichnung "axiales Verstärkungselement 9" ist so zu verstehen, dass das axiale Verstärkungselement 9 axiale Kräfte aufnehmen kann. Das bedeutet jedoch nicht notwendigerweise, dass das axiale Verstärkungselement 9 senkrecht zur axialen Richtung A angeordnet sein muss, so wie es beispielsweise in Fig. 10 und Fig. 11 gezeigt ist. Es sind auch solche Ausführungsformen möglich, bei denen das axiale Verstärkungselement schräg zur axialen Richtung A ausgestaltet ist.

Vorzugsweise ist das axiale Verstärkungselement 9 als eine metallische Platte ausgestaltet, die mit einer Kunststoffbeschichtung vollständig umschlossen ist. Für die metallische Platte ist ein rostfreier Stahl bzw. ein Edelstahl bevorzugt. Für die Kunststoffbeschichtung ist ein chemisch hoch resistenter Kunststoff bevorzugt. Beispiele für solche bevorzugten Kunststoffe sind PTFE, PFA, ECTFE (Ethylenchlortrifluorethylen), ETFE (Ethylen-Tetrofluorethylen), PP (Polypropylen), PE (Polyethylen). Alternativ ist es auch möglich, das axiale Verstärkungselement 9 vollständig aus einem starken bzw. stabilen Kunststoff herzustellen.

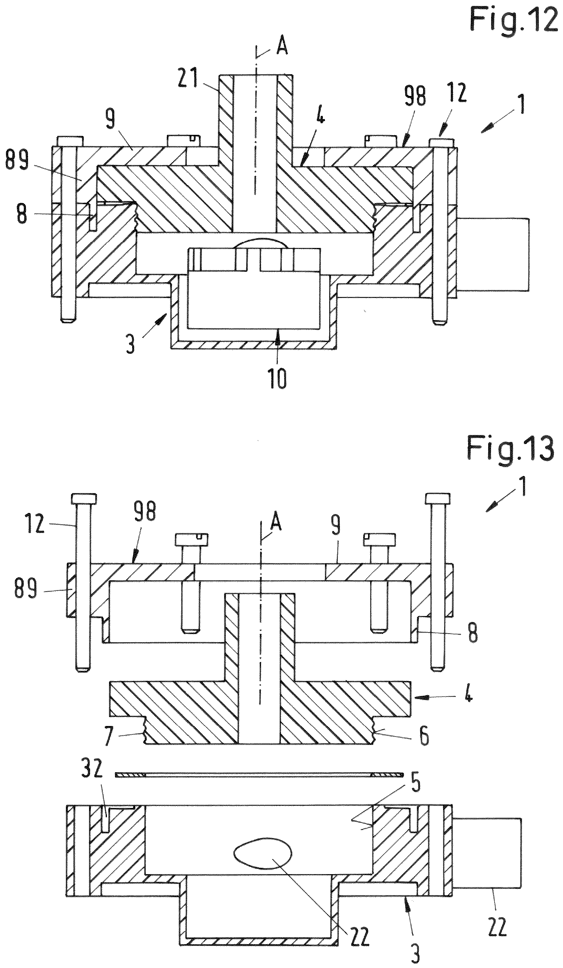

Fig. 12 zeigt in einer zu Fig. 10 analogen Darstellung eine Schnittdarstellung einer Variante des zweiten Ausführungsbeispiels der erfindungsgemässen Pumpeneinheit 1. Zum besseren Verständnis zeigt Fig. 13 diese Variante noch in einer Explosionsdarstellung.

Bei der in den Fig. 12 und 13 gezeigten Variante ist das axiale Verstärkungselement 9 einstückig mit dem radialen Verstärkungselement 8 ausgestaltet. Es ist also ein einstückiges Verstärkungselement 98 vorgesehen, welches sowohl das axiale Verstärkungselement 9 als auch das radiale Verstärkungselement 8 bildet.

Das einstückige Verstärkungselement 98 umfasst das axiale Verstärkungselement 9, welches als ringscheibenförmige Platte ausgestaltet ist, die den Einlass 21 umgibt und auf dem Deckel 4 angeordnet ist, sodass der Deckel 4 zwischen dem axialen Verstärkungselement 9 und dem Bodenteil 3 angeordnet ist. Das einstückige Verstärkungselement 98 umfasst ferner das ringförmige Verstärkungselement 8, welches ringförmig ausgestaltet ist, und sich radial aussenliegend um die beiden Dichtflächen herum erstreckt. Das radiale Verstärkungselement 8 ist in der Umfangsnut 32 im Bodenteil 3 angeordnet Das einstückige Verstärkungselement 98 umfasst ferner einen ringförmigen Verbindungsbereich 89, welcher das axiale Verstärkungselement 9 mit dem radialen Verstärkungselement 8 verbindet. Der ringförmige Verbindungsbereich 89 schliesst sich an den radial äusseren Rand des axialen Verstärkungselements 9 an, und ist so ausgestaltet, dass er den Deckel 4 radial aussenliegend umschliesst und eng an dem Deckel 4 anliegt.

Vorzugsweise ist das einstückige Verstärkungselement 98 mit einem metallischen Grundkörper ausgestaltet, der mit einer Kunststoffbeschichtung vollständig umschlossen ist. Für den metallischen Grundkörper ist ein rostfreier Stahl bzw. ein Edelstahl bevorzugt. Für die Kunststoffbeschichtung ist ein chemisch hoch resistenter Kunststoff bevorzugt. Beispiele für solche bevorzugten Kunststoffe sind PTFE, PFA, ECTFE (Ethylenchlortrifluorethylen), ETFE (Ethylen-Tetrafluorethylen) PP (Polypropylen), PE (Polyethylen). Alternativ ist es auch möglich, das einstückige Verstärkungselement 98 vollständig aus einem starken bzw. stabilen Kunststoff herzustellen.

Die einstückige Ausgestaltung des radialen Verstärkungselements 8 mit dem axialen Verstärkungselement 9 als einstückiges Verstärkungselement 98 hat den Vorteil einer besonders einfachen und damit auch kostengünstigen Herstellung. Ausserdem verleiht das einstückige Verstärkungselement 98 der Pumpeneinheit 1 eine besonders hohe Robustheit, beispielsweise weil der Deckel 4 entlang seiner radial äusseren Umfangsfläche vollständig umfasst und damit gestützt wird, sodass Kriecheffekte noch weiter reduziert werden können. Dies ist insbesondere auch ein Vorteil bei Anwendungen, bei denen Fluide mit einer hohen Temperatur gefördert werden.

Es versteht sich, dass die Variante mit dem einstückigen Verstärkungselement 98 auch für alle anderen Ausführungsbeispiele der erfindungsgemässen Pumpeneinheit 1 geeignet ist.

Fig. 14 zeigt in einer zu Fig. 11 analogen Explosionsdarstellung eine Schnittdarstellung eines dritten Ausführungsbeispiels einer erfindungsgemässen Pumpeneinheit 1.

Im Folgenden wird nur auf die Unterschiede zu den vorangehend beschriebenen Ausführungsbeispielen eingegangen. Gleiche Teile oder von der Funktion her gleichwertige Teile des dritten Ausführungsbeispiels sind mit den gleichen Bezugszeichen bezeichnet wie bei den vorangehend beschriebenen Ausführungsbeispielen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen bezüglich der Ausführungsbeispiele und der Varianten in gleicher Weise oder in sinngemäss gleicher Weise auch für das dritte Ausführungsbeispiel gelten.

Das dritte Ausführungsbeispiel der erfindungsgemässen Pumpeneinheit 1 ist ähnlich ausgestaltet wie das zweite Ausführungsbeispiel (Fig. 11). Jedoch ist bei dem dritten Ausführungsbeispiel die Umfangsnut 32 im Bodenteil des zweiten Ausführungsbeispiels durch eine ringförmige Nut 33 ersetzt, die bezüglich ihrer in radialer Richtung gemessenen Breite deutlich breiter ausgestaltet ist als die Umfangsnut 32 des zweiten Ausführungsbeispiels.

Die ringförmige Nut 33 wird bezüglich von der radialen Richtung von einer radialen Innenwand 331 und einer radialen Aussenwand 332 begrenzt, wobei die radiale Aussenwand 332 radial aussenliegend bezüglich der radialen Innenwand 331 angeordnet ist. Die Breite der ringförmigen Nut 33 ist somit der radiale Abstand zwischen der radialen Innenwand 331 und der radialen Aussenwand 332. Die radiale Innenwand 331 bildet die erste Dichtfläche 5 des Bodenteils 3, wobei die erste Dichtfläche 5 hier als die glatte Dichtfläche ausgestaltet ist.

Der Deckel 4 des dritten Ausführungsbeispiels ist mit einem ringförmigen axialen Endbereich 44 ausgestaltet, der in die ringförmige Nut 33 eingreift. Der ringförmige axiale Endbereich 44 wird bezüglich der radialen Richtung durch eine radiale Innenfläche 441 und durch eine radiale Aussenfläche 442 begrenzt, wobei die radiale Aussenfläche 442 radial aussenliegend bezüglich der radialen Innenfläche 441 angeordnet ist. Die radiale Innenfläche 441 des axialen Endbereichs 44 bildet, die zweite Dichtfläche 6 des Deckels 4 die hier als die Rippenfläche mit den Dichtungsrippen 7 ausgestaltet ist.

Wenn der axiale Endbereich 44 des Deckels 4 mit der ringförmigen Nut 33 des Bodenteils in Eingriff gebracht ist, wirken die von der radialen Innenwand 331 gebildete erste Dichtfläche 5 und die von der radialen Innenfläche 441 gebildete zweite Dichtfläche 6 dichtend zusammen.

Die Breite der ringförmigen Nut 33 ist so bemessen, dass das ringförmige radiale Verstärkungselement 8 in die ringförmige Nut 33 eingreifen kann, sodass das radiale Verstärkungselement 8 bezüglich der radialen Richtung zwischen dem axialen Endbereich 44 und der radialen Aussenwand 332 angeordnet ist.

Die radialen Erstreckungen der ringförmigen Nut 33, des axialen Endbereichs 44 und des radialen Verstärkungselements 8 sind so bemessen, dass ein Presssitz zwischen dem Deckel 4 und dem Bodenteil 3 realisiert ist und die erste Dichtfläche 5 dichtend mit der zweiten Dichtfläche 6 zusammenwirkt.

Gemäss einer Variante des dritten Ausführungsbeispiels der Pumpeneinheit 1, die in der Zeichnung nicht dargestellt ist, ist die erste Dichtfläche 5, also die Dichtfläche 5 des Bodenteils 3 als die Rippenfläche mit den Dichtungsrippen 7 ausgestaltet, und die zweite Dichtfläche 6, also die Dichtfläche 6 des Deckels 4 ist als die glatte Fläche ausgestaltet. Bei dieser Variante ist also die die radiale Innenwand 331 der ringförmigen Nut 33 als die Rippenfläche ausgestaltet und die radiale Innenfläche 441 des axialen Endbereichs 44 ist als die glatte Fläche ausgestaltet.

Fig. 15 zeigt in einer zu Fig. 11 analogen Explosionsdarstellung eine Schnittdarstellung eines vierten Ausführungsbeispiels einer erfindungsgemässen Pumpeneinheit 1.

Im Folgenden wird nur auf die Unterschiede zu den vorangehend beschriebenen Ausführungsbeispielen eingegangen. Gleiche Teile oder von der Funktion her gleichwertige Teile des vierten Ausführungsbeispiels sind mit den gleichen Bezugszeichen bezeichnet wie bei den vorangehend beschriebenen Ausführungsbeispielen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen bezüglich der Ausführungsbeispiele und der Varianten in gleicher Weise oder in sinngemäss gleicher Weise auch für das vierte Ausführungsbeispiel gelten.

Wie bereits vorangehend erwähnt sind für das erfindungsgemässe Pumpengehäuse 1 - und das gilt insbesondere auch für alle hier beschriebenen Ausführungsbeispiele - sowohl Ausführungsformen möglich, bei denen die erste Dichtfläche 5 des Bodenteils 3 als glatte Fläche ausgestaltet ist und die zweite Dichtfläche 6 des Deckels 4 als Rippenfläche mit den Dichtungsrippen 7, als auch Ausführungsformen, bei denen die erste Dichtfläche 5 des Bodenteils 3 als Rippenfläche mit den Dichtungsrippen 7 ausgestaltet ist, und die zweite Dichtfläche 6 des Deckels 4 als glatte Fläche.

Das vierte Ausführungsbeispiel der erfindungsgemässen Pumpeneinheit 1 zeigt mit beispielhaftem Charakter eine Ausgestaltung, bei welcher die erste Dichtfläche 5 als Rippenfläche ausgestaltet ist. Das vierte Ausführungsbeispiel ist ähnlich ausgestaltet wie das zweite Ausführungsbeispiel (Fig. 11). Bei dem vierten Ausführungsbeispiel ist jedoch die erste Dichtfläche 5, also die Dichtfläche des Bodenteils 3 als Rippenfläche mit den Dichtungsrippen 7 ausgestaltet. Die zweite Dichtungsfläche 6, also die Dichtfläche des Deckels 4 ist als glatte Fläche ausgestaltet.

Fig. 16 zeigt in einer zu Fig. 10 analogen Schnittdarstellung ein fünftes Ausführungsbeispiels einer erfindungsgemässen Pumpeneinheit 1.

Im Folgenden wird nur auf die Unterschiede zu den vorangehend beschriebenen Ausführungsbeispielen eingegangen. Gleiche Teile oder von der Funktion her gleichwertige Teile des fünften Ausführungsbeispiels sind mit den gleichen Bezugszeichen bezeichnet wie bei den vorangehend beschriebenen Ausführungsbeispielen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen bezüglich der Ausführungsbeispiele und der Varianten in gleicher Weise oder in sinngemäss gleicher Weise auch für das fünfte Ausführungsbeispiel gelten.

Das fünfte Ausführungsbeispiel (Fig. 16) der erfindungsgemässen Pumpeneinheit 1 ist ähnlich ausgestaltet wie das zweite Ausführungsbeispiel (Fig. 10).

Bei dem fünften Ausführungsbeispiel ist im Deckel 4 eine ringförmige Ausnehmung 45 vorgesehen, die sich radial innenliegend und benachbart zu der zweiten Dichtfläche 6 erstreckt, wobei die ringförmige Ausnehmung 45 an ihrer radialen Aussenseite durch einen ringförmigen Steg 46 von der zweiten Dichtfläche 6 getrennt ist. Die radial innenliegende Begrenzungsfläche 461 des Stegs 46 bildet die radial äussere Begrenzung der Ausnehmung 45. Die radial aussenliegende Begrenzungsfläche des Stegs 46 bildet die zweite Dichtfläche 6, also die Dichtfläche 6 des Deckels 4, die hier als die Rippenfläche mit den Dichtungsrippen 7 ausgestaltet ist. Somit ist die ringförmige Ausnehmung 45 bezüglich der axialen Richtung A auf der gleichen Höhe angeordnet wie die Dichtflächen 5 und 6. Im Betriebszustand der Pumpeneinheit 1 ist natürlich auch die ringförmige Ausnehmung 45 mit dem Fluid gefüllt, sodass der Druck des Fluids eine radial nach aussen gerichtete Kraft auf den Steg 46 ausübt, welche das dichtende Zusammenwirken der ersten Dichtfläche 5 und der zweiten Dichtfläche 6 verstärkt.

Bei allen vorangehend beschriebenen Ausführungsbeispielen und Varianten ist der Auslass des Pumpengehäuses im Bodenteil 3 vorgesehen. Insbesondere ist der Auslass 22 darstellungsgemäss immer unterhalb der Dichtungsrippen 7 angeordnet, d.h. bezüglich der axialen Richtung A ist der Auslass 22 zwischen dem zylindrischen Becher 31 und den beiden Dichtflächen 5 und 6 angeordnet. Dies ist auch die bevorzugte Ausgestaltung, dass also alle Dichtungsrippen 7 oberhalb (gemäss der Darstellung in den Fig. 3, 4, 8-16) des Auslasses 22 angeordnet sind. Es sind aber auch solche Ausführungsformen möglich, bei denen die Trennfläche zwischen dem Deckel 4 und dem Bodenteil 3 bezüglich der axialen Richtung A deutlich näher an dem zylindrischen Becher 31 angeordnet ist, sodass der Auslass 22 darstellungsgemäss oberhalb der Dichtungsrippen 7 liegt. Beispielsweise kann der Auslass 22 im Deckel 4 angeordnet sein).

Fig. 17 zeigt in einer zu Fig. 3 analogen Schnittdarstellung ein sechstes Ausführungsbeispiels einer erfindungsgemässen Pumpeneinheit 1. Zum besseren Verständnis zeigt Fig. 18 das sechste Ausführungsbeispiel der Pumpeneinheit 1 noch in einer Explosionsdarstellung.

Im Folgenden wird nur auf die Unterschiede zu den vorangehend beschriebenen Ausführungsbeispielen eingegangen. Gleiche Teile oder von der Funktion her gleichwertige Teile des fünften Ausführungsbeispiels sind mit den gleichen Bezugszeichen bezeichnet wie bei den vorangehend beschriebenen Ausführungsbeispielen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen bezüglich der Ausführungsbeispiele und der Varianten in gleicher Weise oder in sinngemäss gleicher Weise auch für das fünfte Ausführungsbeispiel gelten.

Das sechste Ausführungsbeispiel der erfindungsgemässen Pumpeneinheit 1 ist ähnlich ausgestaltet wie das erste Ausführungsbeispiel (Fig. 3, Fig. 4). Im Unterschied zu dem ersten Ausführungsbeispiel ist bei dem sechsten Ausführungsbeispiel kein radiales Verstärkungselement 8 vorgesehen. Daher sind auch die bei dem ersten Ausführungsbeispiel vorgesehenen Umfangsnuten 32, 42 (Fig. 4) bei dem sechsten Ausführungsbeispiel nicht vorhanden.

Durch die Erfindung wird ferner die Zentrifugalpumpe 200 zum Fördern eines Fluids mit einer Pumpeneinheit 1 vorgeschlagen, wobei die Pumpeneinheit 1 erfindungsgemäss ausgestaltet ist. Fig. 19 zeigt in einer schematischen Schnittdarstellung ein Ausführungsbeispiel einer erfindungsgemässen Zentrifugalpumpe 200. Die Zentrifugalpumpe 200 umfasst die Antriebseinheit 100, die ein Motorgehäuse umfasst. Das Motorgehäuse ist in Fig. 19 aus Gründen der besseren Übersicht nicht dargestellt. Die Antriebseinheit 100 kann aber beispielsweise in analoger Weise ausgestaltet sein, wie die in Fig. 1 dargestellte Antriebseinheit 100' mit dem Motorgehäuse 120`, wobei in dem Motorgehäuse 120' die Ausnehmung121' vorgesehen ist, in welche der zylindrische Becher 31 des Bodenteils 3 des Pumpengehäuses 1 eingesetzt wird.

In dem Motorgehäuse ist ein Stator 102 angeordnet, der mit dem Rotor 10 einen elektromagnetischen Drehantrieb zum Rotieren des Rotors 10 um die axiale Richtung A bildet, wobei die Antriebseinheit 100 zur berührungslosen magnetischen Lagerung des Rotors 10 ausgestaltet ist.

Vorzugsweise ist der Stator 102 als Lager- und Antriebsstator ausgestaltet, mit welchem der Rotor 10 berührungslos magnetisch antreibbar und berührungslos magnetisch bezüglich des Stators 102 lagerbar ist, wobei der Rotor bezüglich der axialen Richtung A passiv magnetisch stabilisiert ist, und in der zur axialen Richtung senkrechten radialen Ebene E aktiv magnetisch, d.h. ansteuerbar, gelagert ist.

Besonders bevorzugt ist der elektromagnetische Drehantrieb mit dem Rotor 10 und dem Stator 102 als Tempelmotor ausgestaltet ist, wobei der Stator 102 eine Mehrzahl von Spulenkernen 125 aufweist, von denen jeder einen Längsschenkel 126 umfasst, welcher sich von einem ersten Ende in axialer Richtung A bis zu einem zweiten Ende erstreckt, sowie einen Querschenkel 127, welcher an dem zweiten Ende des Längsschenkels 126 und in der radialen Ebene E angeordnet ist. Der Querschenkel 127 erstreckt sich von dem Längsschenkel 126 in radialer Richtung nach innen auf den Rotor 10 zu.

Alle ersten Enden der Längsschenkel 126 - also die darstellungsgemäss unteren Enden - sind durch einen Rückschluss 122 zum Führen des magnetischen Flusses miteinander verbunden.

Die Spulenkerne 125 sind bezüglich der Umfangsrichtung um den Rotor 10 herum angeordnet sind, sodass der Rotor 10 zwischen den Querschenkeln 127 der Spulenkerne 125 angeordnet ist. An jedem Längsschenkel 126 ist mindestens eine konzentrierte Wicklung 160 vorgesehen ist, welche den jeweiligen Längsschenkel 126 umgibt.

Mit den konzentrierten Wicklungen 160 werden die für den magnetischen Antrieb und die magnetische Lagerung des Rotors 10 notwendigen elektromagnetischen Drehfelder erzeugt. Mit diesen konzentrierten Wicklungen 160 werden im Betriebszustand also diejenigen elektromagnetischen Drehfelder erzeugt, mit welchen in an sich bekannter Weise ein Drehmoment auf den Rotor 10 bewirkt wird, und mit welchen eine beliebig einstellbare Querkraft in radialer Richtung auf den Rotor 10 ausübbar ist, sodass die radiale Position des Rotors 10, also seine Position in der zur axialen Richtung A senkrechten radialen Ebene E, aktiv steuerbar bzw. regelbar ist. Bezüglich dreier weiterer Freiheitsgrade, nämlich seiner Position in axialer Richtung und Verkippungen bezüglich der zur Solldrehachse senkrechten radialen Ebene E (zwei Freiheitsgrade), ist der Rotor 10 passiv magnetisch, das heisst nicht ansteuerbar, durch Reluktanzkräfte gelagert bzw. stabilisiert.

## Patentansprüche

1. Pumpeneinheit für eine Zentrifugalpumpe zum Fördern eines Fluids, umfassend ein Pumpengehäuse (2) mit einem Einlass (21) und mit einem Auslass (22) für das Fluid, und einen in dem Pumpengehäuse (2) angeordneten Rotor (10) zum Fördern des Fluids, der um eine axiale Richtung (A) rotierbar ist, wobei die Pumpeneinheit für eine berührungslos magnetische Lagerung des Rotors (10) und für einen berührungslos magnetischen Antrieb des Rotors (10) ausgestaltet ist, wobei das Pumpengehäuse (2) ein Bodenteil (3) und einen Deckel (4) zum Verschliessen des Bodenteils (3) aufweist, wobei das Bodenteil (3) einen zylindrischen Becher (31) zur Aufnahme des Rotors (10) und eine im Wesentlichen ringförmige erste Dichtfläche (5) zum dichtenden Zusammenwirken mit dem Deckel (4) aufweist, und wobei der Deckel (4) eine im Wesentlichen ringförmige zweite Dichtfläche (6) aufweist, die zum dichtenden Zusammenwirken mit der ersten Dichtfläche (5) ausgestaltet ist, **dadurch gekennzeichnet, dass** eine der beiden Dichtflächen (5, 6) als Rippenfläche mit mindestens einer radialen Dichtungsrippe (7) ausgestaltet ist, die sich in Umfangsrichtung entlang der gesamten Dichtfläche (5, 6) erstreckt, während die andere der beiden Dichtflächen (6, 5) als glatte Fläche ausgestaltet ist.

2. Pumpeneinheit nach Anspruch 1, bei welcher ein radiales Verstärkungselement (8) vorgesehen ist, welches ringförmig ausgestaltet ist, und sich radial aussenliegend um die beiden Dichtflächen (5, 6) herum erstreckt.

3. Pumpeneinheit nach einem der vorangehenden Ansprüche, bei welcher die Rippenfläche mehrere radiale Dichtungsrippen (7) umfasst, von denen sich jede in Umfangsrichtung entlang der gesamten Rippenfläche erstreckt, wobei die Dichtungsrippen (7) bezüglich der axialen Richtung (A) benachbart zueinander angeordnet sind, und zwischen zwei benachbarten Dichtungsrippen (7) jeweils ein Tal (71) vorgesehen ist, wobei jedes Tal (71) einen von Null verschiedenen radialen Abstand (R) von der glatten Fläche hat, und wobei jede Dichtungsrippe (7) eine in radialer Richtung gemessene Höhe (H) aufweist, die grösser ist als der radiale Abstand (R) des Tals (71) von der glatten Fläche.

4. Pumpeneinheit nach einem der Ansprüche 2-3, bei welchem das radiale Verstärkungselement (8) sowohl in den Deckel (4) als auch in das Bodenteil (3) eingreift.

5. Pumpeneinheit nach einem der vorangehenden Ansprüche, wobei die glatte Fläche kegelstumpfförmig ausgestaltet ist.

6. Pumpeneinheit nach einem der vorangehenden Ansprüche, wobei bezüglich der radialen Richtung zwischen den beiden Dichtflächen (5, 6) einerseits und dem radialen Verstärkungselement (8) andererseits eine ringförmige Sicherheitsdichtung (20) angeordnet ist, welche im Fehlerfall ein Austreten des Fluids zwischen dem Bodenteil (3) und dem Deckel (4) verhindert.

7. Pumpeneinheit nach einem der vorangehenden Ansprüche, wobei auf dem Deckel (4) ein axiales Verstärkungselement (9) zum Aufnehmen von in axialer Richtung (A) wirkenden Kräften vorgesehen ist, wobei das axiale Verstärkungselement (9) so angeordnet ist, dass sich der Deckel (4) bezüglich der axialen Richtung (A) zwischen dem axialen Verstärkungselement (9) und dem Bodenteil (3) befindet.

8. Pumpeneinheit nach Anspruch 6, wobei das axiale Verstärkungselement (9) einstückig mit dem radialen Verstärkungselement (8) ausgestaltet ist.

9. Pumpeneinheit nach einem der vorangehenden Ansprüche, wobei das Bodenteil (3) eine ringförmige Nut (33) umfasst, die von einer radialen Innenwand (331) und von einer radialen Aussenwand (332) begrenzt wird, wobei der Deckel (4) zum dichtenden Eingreifen in die Nut (33) ausgestaltet ist, und wobei die radiale Innenwand (331) oder die radiale Aussenwand (332) die erste Dichtfläche (5) bildet.

10. Pumpeneinheit nach einem der vorangehenden Ansprüche, wobei im Deckel (3) eine ringförmige Ausnehmung (45) vorgesehen ist, die sich radial innenliegend und benachbart zu der zweiten Dichtfläche (6) erstreckt, wobei die ringförmige Ausnehmung (45) durch einen ringförmigen Steg (46) von der zweiten Dichtfläche (6) getrennt ist, sodass im Betriebszustand der Druck des Fluids auf den Steg (46) das dichtende Zusammenwirken der ersten Dichtfläche (5) und der zweiten Dichtfläche (6) verstärkt.

11. Pumpeneinheit nach einem der vorangehenden Ansprüche, wobei die erste Dichtfläche (5) als die glatte Dichtfläche ausgestaltet ist, und die zweite Dichtfläche (6) als die Rippenfläche ausgestaltet ist

12. Pumpeneinheit nach einem der Ansprüche 2-11, wobei das radiale Verstärkungselement (8) als metallischer Ring ausgestaltet ist, der mit einer Beschichtung aus einem Kunststoff gekapselt ist.

13. Zentrifugalpumpe zum Fördern eines Fluids mit einer Pumpeneinheit (1), die nach einem der vorangehenden Ansprüche ausgestaltet ist, und mit einer Antriebseinheit (100), die ein Motorgehäuse umfasst, in welchem ein Stator (102) angeordnet ist, der mit dem Rotor(10) einen elektromagnetischen Drehantrieb zum Rotieren des Rotors (10) um die axiale Richtung (A) bildet, wobei die Antriebseinheit (100) zur berührungslosen magnetischen Lagerung des Rotors (10) ausgestaltet ist.

14. Zentrifugalpumpe nach Anspruch 13, wobei der Stator (102) als Lager- und Antriebsstator ausgestaltet ist, mit welchem der Rotor (10) berührungslos magnetisch antreibbar und berührungslos magnetisch bezüglich des Stators (2) lagerbar ist, wobei der Rotor (10) bezüglich der axialen Richtung (A) passiv magnetisch stabilisiert ist, und in einer zur axialen Richtung (A) senkrechten radialen Ebene (E) aktiv magnetisch gelagert ist, wobei das Motorgehäuse in einer axialen Stirnfläche eine Ausnehmung aufweist, in welche der zylindrische Becher (31) des Pumpengehäuses (1) einsetzbar ist.

15. Zentrifugalpumpe nach Anspruch 14, bei welchem der elektromagnetische Drehantrieb als Tempelmotor ausgestaltet ist, wobei der Stator (102) eine Mehrzahl von Spulenkernen (125) aufweist, von denen jeder einen Längsschenkel (125) umfasst, welcher sich von einem ersten Ende in axialer Richtung (A) bis zu einem zweiten Ende erstreckt, sowie einen Querschenkel (127), welcher an dem zweiten Ende des Längsschenkels (126) und in der radialen Ebene (E) angeordnet ist, und welcher sich von dem Längsschenkel (126) in radialer Richtung erstreckt, wobei die Spulenkerne (125) bezüglich der Umfangsrichtung um den Rotor (10) herum angeordnet sind, sodass der Rotor (10) zwischen den Querschenkeln (127) der Spulenkerne (125) angeordnet ist, und wobei an jedem Längsschenkel (125) mindestens eine konzentrierte Wicklung (160) vorgesehen ist, welche den jeweiligen Längsschenkel (126) umgibt.
